# EUROPÄISCHE PATENTANMELDUNG

(11) **EP 4 049 756 A1**
(43) Veröffentlichungstag der Anmeldung: **31.08.2022**
(21) Anmeldenummer: 21158992.4
(22) Anmeldetag: 24.02.2021
(51) Int. Cl.: B01L 3/00, G01N 21/03, A61B 10/00

(54) **BEHÄLTER FÜR EINE PROBE UND VERFAHREN ZUR UNTERSUCHUNG EINER PROBE MIT DEM BEHÄLTER**

(71) Anmelder: Paul Marienfeld GmbH & Co. KG, 97922 Lauda-Königshofen (DE)
(72) Erfinder: Marienfeld, Gerd, 97922 Lauda-Königshofen (DE)
(74) Vertreter: Ter Meer Steinmeister & Partner

(57) **Zusammenfassung**

Die Erfindung betrifft einen Behälter für eine Probe. Um eine optische oder spektroskopische Untersuchung einer in dem Behälter gelagerten Probe zu verbessern, wird ein Behälter vorgeschlagen, umfassend einen Bodenabschnitt, wobei eine Behälterachse senkrecht zu dem Bodenabschnitt orientiert ist; einen ersten Wandabschnitt, wobei sich der erste Wandabschnitt an den Bodenabschnitt anschließt; und einen zweiten Wandabschnitt, wobei sich der zweite Wandabschnitt an den ersten Wandabschnitt anschließt, und wobei der zweite Wandabschnitt gegenüber der Behälterachse um zumindest 20 ° geneigt ist.

## Beschreibung

Die Offenbarung betrifft einen Behälter für eine Probe, insbesondere für eine biologische Probe. Weiterhin betrifft die Offenbarung ein Verfahren zur Untersuchung einer Probe sowie eine Verwendung eines Behälters zur Lagerung einer Probe während einer Untersuchung.

Zur Untersuchung von Gewebeproben kann Gewebe aus dem Körper von Lebewesen (z.B. Menschen oder Tieren) entnommen werden und dieses bearbeitet und dann bspw. optisch untersucht werden. Die Bearbeitung kann ein Dehydrieren des Gewebes und ein Clearing umfassen, wobei während des Clearing-Prozesses Alkohol, der oft zur Dehydrierung des Gewebes eingesetzt wird, entfernt werden kann. Die bearbeitete Probe kann in wenige Mikrometer starke Scheiben zerteilt werden, die dann auf einem Objektträger angeordnet oder befestigt wird. Diese Probe kann auch eingefärbt werden. Der Objektträger mit der Probe kann dann in einen Behälter eingebracht werden und kann mit einer Flüssigkeit bedeckt werden. Der Behälter kann mit einer (dünnen) Glasscheibe bedeckt werden. Die zerteilte und eingefärbte Gewebeprobe kann dann optisch untersucht werden, beispielsweise durch Durchlicht- oder Fluoreszenz-Mikroskopie. Die Untersuchung findet außerhalb des Körpers des Lebewesens statt. Durch diese Untersuchung kann jedoch keine Information über die dreidimensionale Position von Abschnitten der Probe gewonnen werden, d.h. es ist nicht möglich, eine dreidimensionale Position der Zellen innerhalb des Gewebes oder Bestandteile des Gewebes zu bestimmen.

Wird eine Flüssigkeit in einem Behälter gelagert, kommt es zu einer Wechselwirkung zwischen der Flüssigkeit und der Oberfläche der Behälterwand. In Abhängigkeit der Flüssigkeit und des Materials der Behälterwand bildet sich in dem Kontaktbereich zwischen Flüssigkeit und Behälterwand eine Wölbung der Flüssigkeitsoberfläche (konkav oder konvex) aus. Die gewölbte Flüssigkeitsoberfläche - auch konkaver Meniskus genannt - wirkt als Linse, durch die eine Untersuchung einer Probe in dem Behälter, bedeckt von der Flüssigkeit, gestört bzw. verfälscht werden kann.

Der Erfindung liegt daher die Aufgabe zugrunde einen Behälter bereitzustellen, durch den eine optische oder spektroskopische Untersuchung einer in dem Behälter gelagerten Probe verbessert wird, insbesondere wenn die Probe mit einer Flüssigkeit bedeckt ist und wenn die Strahlungsquelle und der Strahlungsempfänger nicht auf einer Linie mit der Probe liegen.

Eine weitere Aufgabe der Erfindung liegt darin einen Behälter bereitzustellen, durch den eine Untersuchung mehrerer Ebenen einer Probe in dem Behälter ermöglicht wird, wodurch eine dreidimensionale Untersuchung durchführbar ist.

Eine noch weitere Aufgabe liegt in der kostengünstigen Herstellbarkeit des Behälters.

Gelöst wird die Aufgabe durch die Gegenstände und Verfahren der unabhängigen Ansprüche. Vorteilhafte Ausgestaltungen sind in den abhängigen Ansprüchen spezifiziert.

Ein Behälter für eine Probe umfasst einen Bodenabschnitt, einen ersten Wandabschnitt und einen zweiten Wandabschnitt. Eine Behälterachse ist senkrecht zu dem Bodenabschnitt orientiert oder gelegen. Der erste Wandabschnitt schließt sich an den Bodenabschnitt an. Der zweite Wandabschnitt schließt sich an den ersten Wandabschnitt an. Der zweite Wandabschnitt ist gegenüber der Behälterachse um zumindest 20 ° geneigt. D.h. der Behälter hat eine ebene Bodenfläche, an die sich die Bodenfläche umschließend ein erster Wandabschnitt anschließt, der nach oben absteht. Der erste Wandabschnitt bildet somit mit der Bodenfläche einen ersten Aufnahmeraum. An diesen erster Wandabschnitt schließt sich ein zweiter Wandabschnitt an, der eine andere Neigung als der erster Wandabschnitt aufweist. Der zweite Wandabschnitt umschließt somit einen zweiten Aufnahmeraum oberhalb des ersten Aufnahmeraums.

Auch kann ein Behälter für eine Probe einen Bodenabschnitt, einen ersten Wandabschnitt und einen zweiten Wandabschnitt umfassen. Eine Behälterachse ist senkrecht zu dem Bodenabschnitt orientiert oder gelegen. Der erste Wandabschnitt schließt sich an den Bodenabschnitt an und der zweite Wandabschnitt schließt sich an den ersten Wandabschnitt an. Eine Querschnittsfläche des Behälters auf halber Höhe des ersten Wandabschnitts kann kleiner sein (um zumindest 5 % oder zumindest 10 %) als die Querschnittsfläche des Behälters auf halber Höhe des zweiten Wandabschnitts. Die halbe Höhe kann die Hälfte der Gesamthöhe des jeweiligen Wandabschnitts in Richtung der Behälterachse sein.

D.h. der Behälter hat eine ebene Bodenfläche, an die sich die Bodenfläche umschließend ein erster Wandabschnitt anschließt, der nach oben absteht. Der erste Wandabschnitt bildet somit mit der Bodenfläche einen ersten Aufnahmeraum. An diesen erster Wandabschnitt schließt sich ein zweiter Wandabschnitt an, der eine andere Neigung als der erster Wandabschnitt aufweist. Der zweite Wandabschnitt umschließt somit einen zweiten Aufnahmeraum oberhalb des ersten Aufnahmeraums.

Durch die Ausgestaltung des zweiten Wandabschnitts kann eine Flüssigkeit so in den Behälter eingebracht werden, dass eine für eine optische oder spektroskopische Untersuchung störende Steigung der Flüssigkeitsoberfläche außerhalb des betrachteten Bereichs liegt. Wie oben beschrieben, kann die Probe auf einem Objektträger angehaftet werden, der dann in den Behälter eingebracht wird.

In einer Ebene, die parallel zu dem Bodenabschnitt oder senkrecht zu der Behälterachse gelegen ist und die durch den ersten Wandabschnitt (zumindest einseitig) begrenzt ist, kann die Flüssigkeitsoberfläche im Wesentlichen (± 5 °) eben sein oder im Wesentlichen (± 5 °) ungewölbt oder im Wesentlichen (± 5 °) parallel zu dem Bodenabschnitt oder im Wesentlichen (± 5 °) senkrecht zu der Behälterachse sein. Dadurch kann die Probe auf dem gesamten Bodenabschnitt positioniert werden, ohne die Untersuchung zu beeinträchtigen. Somit kann die Probe im ersten Aufnahmeraum liegen, und mit Flüssigkeit umgeben werden. Die Flüssigkeit wird bis in den zweiten Aufnahmeraum aufgefüllt. Daher bilden sich mögliche Menisken nur im Randbereich des zweiten Aufnahmeraums und können bei einer Bestrahlung der Probe zur optischen Untersuchung nicht störend wirken.

Die Probe kann eine biologische Probe, beispielsweise eine Gewebeprobe, sein. Die Probe kann ein Stoffgemisch, beispielsweise ein homogenes oder heterogenes Stoffgemisch sein. Die Probe kann ein Reinstoff sein.

Der Bodenabschnitt kann eine Fläche von zumindest 200 mm² aufweisen, insbesondere weist der Bodenabschnitt eine Fläche von zumindest 400 mm² oder zumindest 500 mm² auf. Die Fläche des Bodenabschnitts kann höchstens 5000 mm², bevorzugt höchstens 4000 mm², bevorzugter höchstens 3000 mm², bevorzugter höchstens 2500 mm², betragen. Besonders bevorzugt beträgt die Fläche des Bodenabschnitts zwischen 200 mm² und 5000 mm².

Zumindest die Oberfläche des Bodenabschnitts, die in Richtung des Inneren des Behälters weist, kann über seine gesamte Fläche ebenflächig ausgestaltet sein.

Der erste Wandabschnitt kann einstückig mit dem Bodenabschnitt ausgestaltet sein.

Zwischen dem ersten Wandabschnitt und der Behälterachse kann ein Winkel gebildet sein. Um diesen Winkel kann der erste Wandabschnitt gegenüber der Behälterachse geneigt sein. Der Winkel zwischen der Behälterachse und dem ersten Wandabschnitt kann zumindest 0,5 ° betragen. Bevorzugt beträgt der Winkel zwischen der Behälterachse und dem ersten Wandabschnitt zumindest 1,0 ° oder zumindest 1,5 °. Der Winkel zwischen der Behälterachse und dem ersten Wandabschnitt kann höchstens 50 ° betragen, bevorzugt höchstens 40 °, bevorzugter höchstens 30 °, bevorzugter höchstens 25 °, bevorzugter höchstens 10 °, bevorzugter höchstens 5 °. Speziell liegt der Winkel zwischen der Behälterachse und dem ersten Wandabschnitt zwischen 0,5 °und 50 °, bevorzugt zwischen 0,5 ° und 30 °, bevorzugter zwischen 0,5 ° und 10 °. Besonders bevorzugt liegt der Winkel zwischen der Behälterachse und dem ersten Wandabschnitt zwischen 1,0 ° und 3,0 ° oder zwischen 1,5 ° und 2,5 °.

Der erste Wandabschnitt kann eine Höhe (in Richtung der Behälterachse) von zumindest 5 mm, bevorzugt zumindest 7 mm, bevorzugter zumindest 10 mm, bevorzugter zumindest 15 mm, bevorzugter zumindest 24 mm, aufweisen. Die Höhe des ersten Wandabschnitts kann höchstens 100 mm, bevorzugt höchstens 80 mm, bevorzugter höchstens 60 mm, bevorzugter höchstens 50 mm, bevorzugter höchstens 30 mm, bevorzugter höchstens 25 mm oder 15 mm, betragen. Die Höhe des ersten Wandabschnitts kann zwischen 5 mm und 100 mm, bevorzugt zwischen 7 mm und 60 mm liegen.

Der erste Wandabschnitt kann eine Breite (senkrecht zu Richtung der Behälterachse) von zumindest 5 mm, bevorzugter von zumindest 10 mm, bevorzugt von zumindest 20 mm, bevorzugter von zumindest 30 mm, bevorzugter zumindest 35 mm oder 40 mm, aufweisen. Die Breite des ersten Wandabschnitts kann höchstens 150 mm, bevorzugt höchstens 130 mm, bevorzugter höchstens 110 mm, bevorzugter höchstens 90 mm, bevorzugter höchstens 70 mm, bevorzugter höchstens 50 mm, betragen. Die Breite des ersten Wandabschnitts kann zwischen 5 mm und 150 mm, bevorzugt zwischen 10 mm und 100 mm, bevorzugter zwischen 30 mm und 60 mm, liegen. Die Breite kann eine maximale Breite des ersten Wandabschnitts sein.

Der erste Wandabschnitt kann eine um zumindest 5 mm größere Breite als Höhe aufweisen.

Der erste Wandabschnitt kann ebenflächig ausgestaltet sein.

Der zweite Wandabschnitt kann einstückig mit dem ersten Wandabschnitt ausgebildet sein.

Zwischen dem zweiten Wandabschnitt und der Behälterachse ist ein Winkel gebildet. Der zweite Wandabschnitt ist gegenüber der Behälterachse um diesen Winkel geneigt. Der Winkel kann zumindest 30 °, bevorzugt zumindest 40 °, bevorzugter zumindest 50 °, bevorzugter zumindest 55 °, bevorzugter zumindest 60 °, betragen. Der Winkel zwischen dem zweiten Wandabschnitt und der Behälterachse kann höchstens 90 °betragen. Bevorzugt beträgt der Winkel zwischen dem zweiten Wandabschnitt und der Behälterachse höchsten 80 °, bevorzugter höchstens 75 °. Der Winkel zwischen dem zweiten Wandabschnitt und der Behälterachse kann zwischen 20 ° und 90 ° liegen, bevorzugt zwischen 30 ° und 80 °, bevorzugter zwischen 40 ° und 70 °.

Der zweite Wandabschnitt kann eine Höhe (in Richtung der Behälterachse) von zumindest 0,5 mm, bevorzugt zumindest 1 mm, bevorzugter zumindest 1,5 mm, aufweisen. Die Höhe des zweiten Wandabschnitts kann höchstens 30 mm, bevorzugt höchstens 20 mm, bevorzugter höchstens 15 mm, bevorzugter höchstens 10 mm, bevorzugter höchstens 5 mm, betragen. Die Höhe des zweiten Wandabschnitts kann zwischen 0,5 mm und 30 mm, bevorzugt zwischen 1 mm und 20 mm liegen.

Der zweite Wandabschnitt kann eine Breite (senkrecht zu Richtung der Behälterachse) von zumindest 5 mm, bevorzugter von zumindest 10 mm, bevorzugt von zumindest 20 mm, bevorzugter von zumindest 30 mm, bevorzugter zumindest 40 mm oder 50 mm, aufweisen. Die Breite des zweiten Wandabschnitts kann höchstens 150 mm, bevorzugt höchstens 130 mm, bevorzugter höchstens 110 mm, bevorzugter höchstens 90 mm, bevorzugter höchstens 70 mm, bevorzugter höchstens 60 mm, betragen. Die Breite des zweiten Wandabschnitts kann zwischen 5 mm und 150 mm, bevorzugt zwischen 10 mm und 100 mm, bevorzugter zwischen 40 mm und 70 mm, liegen. Die Breite kann eine maximale Breite des ersten Wandabschnitts sein.

An den zweiten Wandabschnitt kann sich ein dritter Wandabschnitt anschließen. Der dritte Wandabschnitt kann einstückig mit dem zweiten Wandabschnitt ausgestaltet sein. Der dritte Wandabschnitt umschließt somit einen dritten Aufnahmeraum, der oberhalb des zweiten Aufnahmeraums angeordnet ist.

Zwischen dem dritten Wandabschnitt und der Behälterachse kann ein Winkel gebildet sein. Der dritte Wandabschnitt kann gegenüber der Behälterachse um diesen Winkel geneigt sein. Der Winkel kann zumindest 1 °, bevorzugt zumindest 2 °, bevorzugter zumindest 3 °, bevorzugter zumindest 4 °, bevorzugter zumindest 5 °, betragen. Der Winkel zwischen dem dritten Wandabschnitt und der Behälterachse kann höchstens 50 °betragen. Bevorzugt beträgt der Winkel zwischen dem dritten Wandabschnitt und der Behälterachse höchsten 40 °, bevorzugter höchstens 30°, bevorzugter höchstens 20 °, bevorzugter höchstens 10 °, bevorzugter höchstens 8 °. Der Winkel zwischen dem dritten Wandabschnitt und der Behälterachse kann zwischen 1 ° und 50 ° liegen, bevorzugt zwischen 2 ° und 30 °, bevorzugter zwischen 3 ° und 10 °.

Der dritte Wandabschnitt kann eine Höhe (in Richtung der Behälterachse) von zumindest 1 mm, bevorzugt zumindest 5 mm, bevorzugter zumindest 8 mm, aufweisen. Die Höhe des dritten Wandabschnitts kann höchstens 100 mm, bevorzugt höchstens 50 mm, bevorzugter höchstens 30 mm, bevorzugter höchstens 20 mm, bevorzugter höchstens 15 mm, betragen. Die Höhe des dritten Wandabschnitts kann zwischen 1 mm und 100 mm, bevorzugt zwischen 1 mm und 50 mm, bevorzugter zwischen 5 mm und 20 mm, liegen.

Der dritte Wandabschnitt kann eine Breite (senkrecht zu Richtung der Behälterachse) von zumindest 5 mm, bevorzugter von zumindest 10 mm, bevorzugter von zumindest 20 mm, bevorzugter von zumindest 30 mm, bevorzugter zumindest 40 mm oder 50 mm, aufweisen. Die Breite des dritten Wandabschnitts kann höchstens 150 mm, bevorzugt höchstens 130 mm, bevorzugter höchstens 110 mm, bevorzugter höchstens 90 mm, bevorzugter höchstens 80 mm, bevorzugter höchstens 70 mm, betragen. Die Breite des dritten Wandabschnitts kann zwischen 5 mm und 150 mm, bevorzugt zwischen 10 mm und 100 mm, bevorzugter zwischen 40 mm und 70 mm, liegen. Die Breite kann eine maximale Breite des ersten Wandabschnitts sein.

Der Behälter kann eine Öffnung aufweisen, die dem Bodenabschnitt gegenüberliegt. Die Öffnung kann durch den dritten Wandabschnitt oder durch dritte Wandabschnitte definiert sein. Die Öffnung kann eine größere Fläche aufweisen als der Bodenabschnitt. Durch die großflächige Öffnung (bezogen auf den Bodenabschnitt) des Behälters kann sichergestellt werden, dass eine Abbildung am Boden des Behälters die numerische Apertur eines Objektivs oberhalb einer Probe in dem Behälter möglichst optimal ausnützen kann. Durch die Ausgestaltung des Behälters wird von der Probe emittiertes Licht in seinem Weg so wenig wie möglich behindert. Ggf. kann das Objektiv in den Behälter, insbesondere in den dritten Aufnahmeraum, der durch den dritten Wandabschnitt gebildet wird, abgesenkt werden.

Die (Winkel oder Neigungen der) Wandabschnitte der Wandseite oder der Wandseiten kann so gewählt werden, dass eine numerische Apertur des Objektivs ausnutzbar ist, insbesondere möglichst optimal ausnutzbar ist.

Der Rand der Öffnung des Behälters kann eine konstante Höhe (in Richtung der Behälterachse) aufweisen. Eine durch die Öffnung des Behälters definierte Ebene kann parallel zu dem Bodenabschnitt sein.

Der zweite Wandabschnitt kann sich an den ersten Wandabschnitt in Richtung der Behälterachse anschließen. Der dritte Wandabschnitt kann sich an den zweiten Wandabschnitt in Richtung der Behälterachse anschließen.

Der erste Wandabschnitt kann, bezogen auf die Behälterachse, eine unterschiedliche Neigung aufweisen als der zweite Wandabschnitt. Der zweite Wandabschnitt kann, bezogen auf die Behälterachse, eine unterschiedliche Neigung aufweisen als der dritte Wandabschnitt.

Der beschriebene erste Wandabschnitt, zweite Wandabschnitt und/oder dritte Wandabschnitt können Wandabschnitte einer ersten Wandseite des Behälters sein. Die erste Wandseite kann aus dem ersten, zweiten und dritten Wandabschnitt bestehen bzw. diese Wandabschnitte aufweisen.

Der Behälter, insbesondere der Bodenabschnitt, kann eine polygone Grundform mit der ersten Wandseite, einer zweiten Wandseite, einer dritten Wandseite und einer vierten Wandseite sein.

Die zweite Wandseite kann einen ersten Wandabschnitt, einen zweiten Wandabschnitt und einen dritten Wandabschnitt aufweisen oder daraus bestehen. Der erste Wandabschnitt, der zweite Wandabschnitt und der dritte Wandabschnitt der zweiten Wandseite können jeweils gleich sein zu dem ersten Wandabschnitt, dem zweiten Wandabschnitt und dem dritten Wandabschnitt der ersten Wandseite.

Die dritte Wandseite kann einen ersten Wandabschnitt, einen zweiten Wandabschnitt und einen dritten Wandabschnitt aufweisen oder daraus bestehen. Der erste Wandabschnitt, der zweite Wandabschnitt und der dritte Wandabschnitt der dritten Wandseite können jeweils gleich sein zu dem ersten Wandabschnitt, dem zweiten Wandabschnitt und dem dritten Wandabschnitt der ersten Wandseite.

Die vierte Wandseite kann einen ersten Wandabschnitt, einen zweiten Wandabschnitt und einen dritten Wandabschnitt aufweisen oder daraus bestehen. Der erste Wandabschnitt, der zweite Wandabschnitt und der dritte Wandabschnitt der vierten Wandseite können jeweils gleich sein zu dem ersten Wandabschnitt, dem zweiten Wandabschnitt und dem dritten Wandabschnitt der ersten Wandseite.

Die polygone Grundform kann eine rechteckige Grundform, insbesondere eine quadratische Grundform sein. Bevorzugt ist die polygone Grundform rechteckig, aber nicht quadratisch. Ebenso kann die polygone Grundform ein n-Eck sein, wobei n ganzzahlig und größer vier ist.

Die Grundform des Behälters, insbesondere des Bodenabschnitts, kann auch einer Ellipse, insbesondere einem Kreis, mit einer Sehne entsprechen. Dabei kann die Sehne der ersten Wandseite entsprechen.

Ebenso können der erste Wandabschnitt, der zweite Wandabschnitt und der dritte Wandabschnitt der dritten Wandseite jeweils eine unterschiedliche Neigung (bezogen auf die Behälterachse) als der erste Wandabschnitt, der zweite Wandabschnitt und der dritte Wandabschnitt der ersten Wandseite aufweisen.

Der erste Wandabschnitt der dritten Wandseite kann eine größere Neigung gegenüber der Behälterachse aufweisen als der erste Wandabschnitt der ersten Wandseite. Bevorzugt ist der erste Wandabschnitt der dritten Wandseite um zumindest 2 ° oder zumindest 5 ° stärker gegenüber der Behälterachse geneigt als der erste Wandabschnitt der ersten Wandseite.

Der erste Wandabschnitt der dritten Wandseite kann um höchstens 30 °, bevorzugt höchstens 20 °, bevorzugter höchstens 15 °, stärker gegenüber der Behälterachse geneigt als der erste Wandabschnitt der ersten Wandseite.

Der zweite Wandabschnitt der dritten Wandseite kann eine geringere Neigung gegenüber der Behälterachse aufweisen als der zweite Wandabschnitt der ersten Wandseite. Bevorzugt ist der zweite Wandabschnitt der dritten Wandseite um zumindest 2 ° oder zumindest 5 ° geringer gegenüber der Behälterachse geneigt als der zweite Wandabschnitt der ersten Wandseite.

Der zweite Wandabschnitt der dritten Wandseite kann um höchstens 30 °, bevorzugt höchstens 20 °, bevorzugter höchstens 15 °, geringer gegenüber der Behälterachse geneigt als der zweite Wandabschnitt der ersten Wandseite.

Der erste Wandabschnitt, der zweite Wandabschnitt und der dritte Wandabschnitt der zweiten Wandseite können jeweils eine unterschiedliche Neigung (bezogen auf die Behälterachse) als der erste Wandabschnitt, der zweite Wandabschnitt und der dritte Wandabschnitt der ersten Wandseite aufweisen.

Der erste Wandabschnitt der zweiten Wandseite kann eine größere Neigung gegenüber der Behälterachse aufweisen als der erste Wandabschnitt der ersten Wandseite. Bevorzugt ist die Neigung des ersten Wandabschnitts der zweiten Wandseite gegenüber der Behälterachse um zumindest 1 ° oder zumindest 2 ° größer als die Neigung des ersten Wandabschnitts der ersten Wandseite gegenüber der Behälterachse.

Der erste Wandabschnitt der zweiten Wandseite kann um höchstens 20 °, bevorzugt höchstens 10 °, bevorzugter höchstens 5 °, größer gegenüber der Behälterachse geneigt sein als der erste Wandabschnitt der ersten Wandseite.

Ebenso kann der zweite Wandabschnitt der zweiten Wandseite eine geringere Neigung gegenüber der Behälterachse aufweisen als der zweite Wandabschnitt der ersten Wandseite. Bevorzugt ist die Neigung des zweiten Wandabschnitts der zweiten Wandseite gegenüber der Behälterachse um zumindest 1 ° oder zumindest 2 ° geringer als die Neigung des zweiten Wandabschnitts der ersten Wandseite gegenüber der Behälterachse.

Der zweite Wandabschnitt der zweiten Wandseite kann um höchstens 20 °, bevorzugt höchstens 10 °, bevorzugter höchstens 5 °, geringer gegenüber der Behälterachse geneigt sein als der erste Wandabschnitt der ersten Wandseite.

Der erste Wandabschnitt, der zweite Wandabschnitt und der dritte Wandabschnitt der vierten Wandseite können jeweils eine unterschiedliche Neigung (bezogen auf die Behälterachse) als der erste Wandabschnitt, der zweite Wandabschnitt und der dritte Wandabschnitt der ersten Wandseite aufweisen.

Der erste Wandabschnitt der vierten Wandseite kann eine größere Neigung gegenüber der Behälterachse aufweisen als der erste Wandabschnitt der ersten Wandseite. Bevorzugt ist die Neigung des ersten Wandabschnitts der vierten Wandseite gegenüber der Behälterachse um zumindest 1 ° oder zumindest 2 ° größer als die Neigung des ersten Wandabschnitts der ersten Wandseite gegenüber der Behälterachse.

Der erste Wandabschnitt der vierten Wandseite kann um höchstens 20 °, bevorzugt höchstens 10 °, bevorzugter höchstens 5 °, größer gegenüber der Behälterachse geneigt sein als der erste Wandabschnitt der ersten Wandseite.

Der zweite Wandabschnitt der vierten Wandseite kann eine geringere Neigung gegenüber der Behälterachse aufweisen als der zweite Wandabschnitt der ersten Wandseite. Bevorzugt ist die Neigung des zweiten Wandabschnitts der vierten Wandseite gegenüber der Behälterachse um zumindest 1 ° oder zumindest 2 ° geringer als die Neigung des zweiten Wandabschnitts der ersten Wandseite gegenüber der Behälterachse.

Der zweite Wandabschnitt der vierten Wandseite kann um höchstens 20 °, bevorzugt höchstens 10 °, bevorzugter höchstens 5 °, geringer gegenüber der Behälterachse geneigt sein als der erste Wandabschnitt der ersten Wandseite.

Ein Behälter für eine Probe kann einen Bodenabschnitt und einen ersten Wandabschnitt umfassen. Eine Behälterachse ist senkrecht zu dem Bodenabschnitt orientiert oder gelegen. Der erste Wandabschnitt schließt sich an den Bodenabschnitt an. Gegenüber der Behälterachse ist der erste Wandabschnitt um 1,9 ° ±0,7 ° oder ein n-faches davon, wobei n ganzzahlig und größer 1 ist, geneigt.

Die Neigung der Behälterachse zum der ersten Wandabschnitt um 1,9 ° ±0,7 ° oder um ein n-faches davon, wobei n ganzzahlig und größer 1 ist, kann auch auf alle hierin beschriebenen Behälter angewendet werden.

Die Neigung des ersten Wandabschnitt um 1,9 ° ±0,7 ° definiert einen Bereich von 1,2 ° bis 2,6 °. Ein ganzzahlig n-faches mit n > 1 davon entspricht einer Multiplikation eines Wertes in dem Bereich mit n. Beispielsweise liegt 1,5 ° in dem Bereich 1,9 ° ±0,7 ° und n kann gleich 3 sein, sodass sich eine Neigung von 4,5 ° ergibt oder 2,2 ° liegt in dem Bereich 1,9 ° ±0,7 ° und n kann gleich 3 sein, sodass sich eine Neigung von 6,6 ° ergibt.

Bevorzugt ist der erste Wandabschnitt gegenüber der Behälterachse um 1,9 ° ±0,5 ° oder ein n-faches davon, wobei n ganzzahlig und größer 1 ist, geneigt. Bevorzugter ist der erste Wandabschnitt gegenüber der Behälterachse um 1,9 ° ±0,3 °, oder ein n-faches davon, wobei n ganzzahlig und größer 1 ist, geneigt. Bevorzugter ist der erste Wandabschnitt gegenüber der Behälterachse um 1,9 ° ±0,15 °, oder ein n-faches davon, wobei n ganzzahlig und größer 1 ist, geneigt.

Der Behälter kann ein spritzgegossener Behälter sein. Der Behälter kann durch Spritzguss hergestellt worden sein.

Der Behälter kann vollständig einstückig hergestellt sein.

Vorzugsweise ist das Material des Behälters transparent. Der Behälter kann vollständig transparent sein. Alle Wandseiten des Behälters können transparent sein.

Der Behälter kann einen Thermoplast umfassen oder aus einem Thermoplast bestehen.

Der Behälter kann aus einem Material bestehen, das für eine Herstellung mittels Spritzguss geeignet ist.

Eine Öffnung des Behälters kann durch den dritten Wandabschnitt oder die dritten Wandabschnitte definiert sein.

Der Behälter kann eine Wandstärke zwischen 0,5 mm und 5,0 mm, bevorzugt zwischen 1,0 mm und 4,0 mm, bevorzugter zwischen 1,0 mm und 2,0 mm, aufweisen.

Der Behälter kann eine Höhe von weniger als 150 mm, bevorzugt weniger als 120 mm, bevorzugter weniger als 100 mm, bevorzugter weniger als 80 mm, bevorzugter weniger als 50 mm, aufweisen.

Der Behälter kann ein Volumen von weniger als 100 ml, bevorzugt weniger als 75 ml, bevorzugter weniger als 50 ml, bevorzugter weniger als 30 ml, aufnehmen. Bevorzugt kann der Behälter ein Volumen von zumindest 5 ml, insbesondere von zumindest 10 ml, aufnehmen. Insbesondere kann der Behälter ein Volumen zwischen 5 ml und 50 ml aufnehmen.

Die dritte Wandseite kann der ersten Wandseite gegenüberliegen. Die vierte Wandseite kann der zweiten Wandseite gegenüberliegen.

Der Behälter kann ein Polymer (Thermoplast) umfassen oder aus dem Polymer bestehen. Das Polymer kann ein Copolymer, insbesondere ein Block-Copolymer, sein.

Das Polymer kann Einheiten umfassen, die durch eine (vollständige) Hydrierung von Polystyrol-Einheiten erhalten wurden. Das Polymer kann Polycyclohexylethen-Einheiten umfassen.

Das Polymer kann Einheiten umfassen, die durch eine (vollständige) Hydrierung von Polybutadien-Einheiten erhalten wurden. Das Polymer kann Ethylen-1-Buten-CopolymerEinheiten umfassen.

Das Polymer kann durch eine (vollständige) Hydrierung eines Styrol-Butadien-Copolymers erhalten worden oder hergestellt worden sein.

Das Polymer kann ein Block-Copolymer, insbesondere ein cyclisches Block-Copolymer, sein.

Das Polymer kann transparent sein, insbesondere in einem Wellenlängenbereich von 380 nm bis 780 nm.

Ein Abschnitt des Behälters kann aus einem Material bestehen, bevorzugt besteht der (gesamte) Behälter aus dem Material.

Das Material kann eine Dichte von höchstens 1,00 g cm⁻³, bevorzugt von höchstens 0,98 g cm⁻³, bevorzugter von höchstens 0,96 g cm⁻³, besonders bevorzugt zwischen 0,92 g cm⁻³ und 0,96 g cm⁻³, aufweisen. Die Dichte kann bestimmt werden nach ASTM D792.

Das Material kann eine Wasseraufnahme von weniger als 5 %, bevorzugt von weniger als 2,0 %, bevorzugter von weniger als 0,5 % aufweisen. Die Wasseraufnahme kann bestimmt werden nach ASTM D570.

Das Material kann eine Schmelzflussrate von zumindest 1,0 cm³ 10 min⁻¹, bevorzugt von zumindest 7,0 cm³ 10 min⁻¹, bevorzugter von zumindest 100,0 cm³ 10 min⁻¹, bevorzugter zwischen 2,0 cm³ 10 min⁻¹ und 250,0 cm³ 10 min⁻¹, aufweisen. Insbesondere liegt die Schmelzflussrate des Materials zwischen 5,0 cm³ 10 min⁻¹ und 20,0 cm³ 10 min⁻¹. Die Schmelzflussrate kann bestimmt werden nach ASTM D1238 bei einem Gewicht von 2,6 kg und einer Temperatur von 260 °C.

Das Material kann eine Transmission von zumindest 50 %, bevorzugt zumindest 70 %, bevorzugter zumindest 85 %, bevorzugter zumindest 90 %, aufweisen. Die Transmission bei 3 mm kann bestimmt werden nach ASTM D1003.

Das Material kann eine Transmission bei einer Wellenlänge von 488 nm und bei einer Wellenlänge von 638 nm von zumindest 50 %, bevorzugt zumindest 60 %, bevorzugter zumindest 70 %, bevorzugter zwischen 80 % und 85 % aufweisen.

Das Material kann eine Trübung von weniger als 5,0 %, bevorzugt von weniger als 3,0 %, bevorzugter von weniger als 1,5 %, bevorzugter von weniger als 1,0 , aufweisen. Die Trübung bei 3 mm kann bestimmt werden nach ASTM D1003.

Das Material kann einen Refraktionsindex von weniger als 5,0, bevorzugt von weniger als 3,0, bevorzugter von weniger als 2,5, bevorzugter von weniger als 2,0, bevorzugter von weniger als 1,6, bevorzugter zwischen 1,46 und 1,56, aufweisen.

Das Material kann eine Vicat-Erweichungstemperatur von zumindest 70 °C, bevorzugt zumindest 85 °C, bevorzugter zumindest 100 °C, bevorzugter zwischen 90 °C und 130 °C, aufweisen. Insbesondere liegt die Vicat-Erweichungstemperatur zwischen 118 °C und 128 °C. Die Vicat-Erweichungstemperatur kann nach ASTM D1525 bei 1 kg und 50 °C h⁻¹, bestimmt werden.

Das Material kann eine Wärmeformbeständigkeitstemperatur von zumindest 50 °C, bevorzugt zumindest 60°C, bevorzugter zumindest 75 °C, bevorzugter zwischen 60°C und 115 °C, aufweisen. Insbesondere liegt die Wärmeformbeständigkeitstemperatur zwischen 97 °C und 108 °C. Die Wärmeformbeständigkeitstemperatur kann nach ASTM D648 bei 0,455 MPa und 2 °C min⁻¹ bestimmt werden.

Das Material kann eine Biegefestigkeit von zumindest 30 MPa, bevorzugt zumindest 40 MPa, bevorzugter zumindest 50 MPa, bevorzugter zwischen 40 MPa und 80 MPa, aufweisen. Insbesondere liegt die Biegefestigkeit zwischen 65 MPa und 75 MPa. Die Biegefestigkeit kann nach ASTM D790 bestimmt werden.

Das Material kann ein Biegemodul von zumindest 600 MPa, bevorzugt zumindest 1000 MPa, bevorzugter zumindest 1400 MPa, bevorzugter zwischen 1000 MPa und 3000 MPa, aufweisen. Insbesondere liegt das Biegemodul zwischen 2200 MPa und 2600 MPa. Das Biegemodul kann nach ASTM D790 bestimmt werden.

Das Material kann eine Zugfestigkeit (Y.P. oder B.P.) von zumindest 15 MPa, bevorzugt zumindest 25 MPa, bevorzugter zumindest 30 MPa, bevorzugter zwischen 20 MPa und 50 MPa, aufweisen. Insbesondere liegt die Zugfestigkeit (Y.P. oder B.P.) zwischen 35 MPa und 48 MPa. Die Zugfestigkeit (Y.P. oder B.P.) kann nach ASTM D638 bestimmt werden.

Das Material kann eine Dehnung von zumindest 5 %, bevorzugt zumindest 8 %, bevorzugter zumindest, 10 %, bevorzugter zwischen 8 % und 40 %, aufweisen. Insbesondere liegt die Dehnung des Materials zwischen 20 % und 29 %. Die Dehnung kann nach ASTM D638 bestimmt werden.

Das Material kann eine Izod-Schlagzähigkeit von zumindest 1 kg-cm cm⁻¹, bevorzugt zumindest 1,5 kg-cm cm⁻¹, bevorzugter zumindest 2,0 kg-cm cm⁻¹, bevorzugter zwischen 1,5 kg-cm cm⁻¹ und 5,0 kg-cm cm⁻¹, aufweisen. Insbesondere liegt die Izod-Schlagzähigkeit zwischen 2,8 kg-cm cm⁻¹ und 4,4 kg-cm cm⁻¹. Die Izod-Schlagzähigkeit kann nach ASTM D256 bestimmt werden.

Das Material kann chemisch beständig sein gegenüber Methylsalicylat (2-Hydroxybenzoesäuremethylester), Dibenzylether (1,1'-[Oxybis(methylen)]bisbenzol), einer Mischung aus einem Teil Benzylalkohol (Phenylmethanol) und zwei Teilen Benzylbenzoat (Benzoesäurephenylmethylester), einer Mischung aus vier Teilen Benzylbenzoat (Phenoxybenzen, 1,1'-Oxybisbenzol) und einem Teil Diphenylether, Ethylcinnamat (Ethyl-3-phenylprop-2-enoat), und/oder 2,2'-Thiodiethanol (Thiodiglycol, Bis(2-hydroxyethyl)sulfid). Die chemische Beständigkeit kann bestimmt werden, indem ein Probekörper zur Untersuchung der Zugfestigkeit (z.B. nach ASTM D638) aus dem Material oder ein Granulat aus dem Material in die Flüssigkeit (Lösungsmittel) für zwei Tage (48 h oder sieben Tage) bei Raumtemperatur (23 °C) eingetaucht (von der Flüssigkeit bedeckt) wird. Nach den zwei Tagen wird der Gewichtsverlust bestimmt und wenn der Gewichtsverlust weniger als 5,0%, bevorzugt weniger als 1,0 %, beträgt, ist das Material chemisch beständig gegenüber der Flüssigkeit. Auch kann nach den zwei Tagen die Bruchdehnung (z.B. nach ASTM D638) bestimmt werden und wenn die Bruchdehnung um weniger 50 %, bevorzugt weniger als 10 %, gegenüber einem nicht in die Flüssigkeit eingetauchten Probekörper abnimmt, ist das Material chemisch beständig gegenüber der Flüssigkeit.

Das Material kann eine Autofluoreszenz, bezogen auf die Autofluoreszenz von Polystyrol, von höchsten 50 %, bevorzugt höchstens 40 %, bevorzugter höchstens 30 %, bevorzugter höchstens 25 %, aufweisen. Die Autofluoreszenz bei einer Anregungswellenlänge von 350 nm (Probendicke 1 mm) kann von dem Material und von Polystyrol bestimmt werden und die Werte für die bestimmte Autofluoreszenz in ein Verhältnis gesetzt werden (Autofluoreszenz Material / Autofluoreszenz Polystyrol). Die Autofluoreszenz kann in einem Wellenlängenbereich von 400 nm bis 460 nm betrachtet werden. Speziell kann die Autofluoreszenz bei einer Wellenlänge von 420 nm betrachtet werden, wobei die Autofluoreszenz des Materials, bezogen auf die Autofluoreszenz von Polystyrol, höchsten 50 %, bevorzugt höchstens 40 %, bevorzugter höchstens 30 %, bevorzugter höchstens 20 %, bevorzugter höchstens 15 %, beträgt.

Bei einer Anregung in einem Wellenlängenbereich von 400 nm bis 900 nm kann das Material keine (messbare) Autofluoreszenz aufweisen.

Jeder der hierin offenbarten Behälter kann in einem Verfahren zur Untersuchung einer Probe eingesetzt werden. Die Probe kann eine biologische Probe sein. Die Probe wird in dem Behälter platziert. Die Probe wird beleuchtet und eine Emission der Probe wird aufgenommen.

Die Probe kann durch eine Strahlungsquelle, insbesondere durch einen Laser, beleuchtet werden.

Die Emission der Probe kann eine Fluoreszenz sein.

Die Probe kann durch den ersten Wandabschnitt der ersten Wandseite des Behälters beleuchtet werden.

Die Emission der Probe kann im Wesentlichen (±10°) senkrecht zur Beleuchtungsrichtung aufgenommen werden.

Die Emission der Probe kann durch ein Objektiv gesammelt werden.

Jeder der hierin offenbarten Behälter kann für eine Lagerung einer Probe während einer Untersuchung der Probe gelagert werden.

Die Untersuchung kann eine mikroskopische Untersuchung, bevorzugter eine lichtscheibenmikroskopische Untersuchung, bevorzugter eine lichtscheibenfluoreszenzmikroskopischen Untersuchung, sein.

Ausführungsbeispiele der Offenbarung sind in den Figuren dargestellt und werden im Folgenden näher beschrieben.
Figur 1 zeigt eine isometrische Ansicht eines Behälters 100.
Figur 2 zeigt den Behälter 100 in einer Ansicht von unten.
Figur 3 zeigt eine Schnittansicht A-A des Behälters 100 wie in Figur 2 angedeutet.
Figur 4 zeigt eine Schnittansicht B-B des Behälters 100 wie in Figur 2 angedeutet.
Figur 5 zeigt eine isometrische Ansicht eines Behälters 200.
Figur 6 zeigt den Behälter 200 in einer Ansicht von unten.
Figur 7 zeigt eine Schnittansicht C-C des Behälters 200 wie in Figur 6 angedeutet.
Figur 8 zeigt eine Schnittansicht D-D des Behälters 200 wie in Figur 6 angedeutet.
Figur 9 Figur zeigt ein System 400 zum Messen einer Probe.

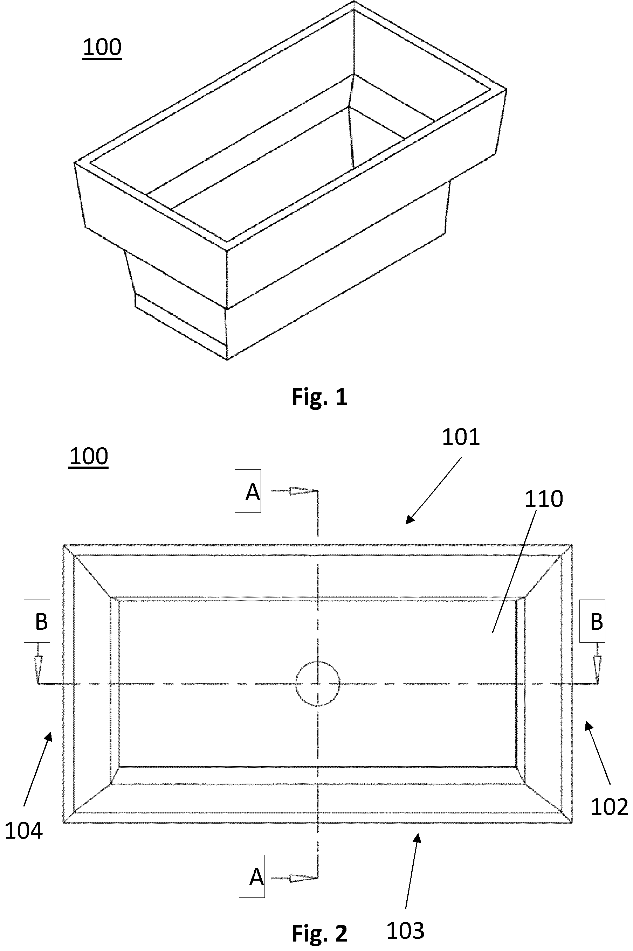

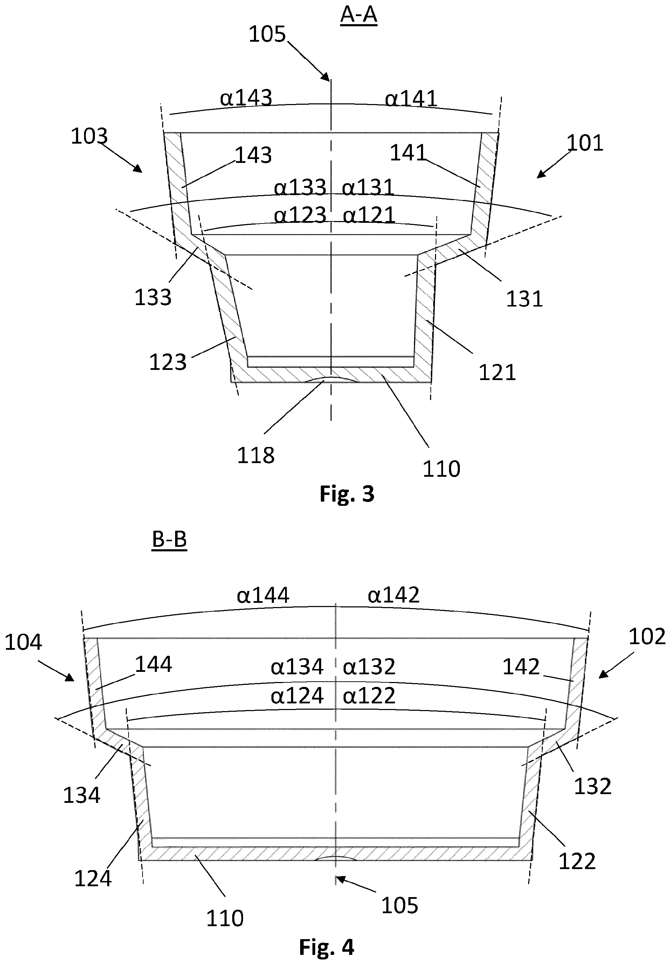

Die Figuren 1 und 2 zeigen einen Behälter 100. In Figur 1 ist der Behälter 100 in einer isometrischen Ansicht und in Figur 2 ist der Behälter 100 in einer Ansicht von unten gezeigt. In Figur 2 sind die Verläufe von zwei Schnitten A-A und B-B angedeutet. Die Schnittansichten A-A und B-B sind in den Figuren 3 und 4 dargestellt.

Der Behälter 100 umfasst einen Bodenabschnitt 110 und eine erste Wandseite 101. Der Behälter 100 kann eine zweite Wandseite 102, eine dritte Wandseite 103 und eine vierte Wandseite 104 umfassen.

Allgemein kann der Behälter 100 eine polygone Grundform aufweisen. Insbesondere kann der Bodenabschnitt 110 des Behälters 100 eine polygone Form aufweisen. Die polygone Grundform oder Form ist insbesondere rechteckig.

Die erste Wandseite 101 und die dritte Wandseite 103 können eine größere Breite aufweisen als die zweite Wandseite 102 und die vierte Wandseite 104. Bevorzugt ist die Breite der ersten Wandseite 101 und der dritten Wandseite 103 um zumindest den Faktor 1,5 größer als die Breite der zweiten Wandseite 102 und der vierten Wandseite 104.

In dem Schnitt A-A der Figur 3 ist die erste Wandseite 101, der Bodenabschnitt 110 und die dritte Wandseite 103 geschnitten dargestellt.

Die innere Oberfläche (in das Innere des Behälters gerichtet) des Bodenabschnitts 110 kann ebenflächig sein. Eine Anspritzkuppe 118 kann in dem Bodenabschnitt 110 so vorgesehen sein, dass trotz der Anspritzkuppe 118 die innere Oberfläche des Bodenabschnitts 110 ebenflächig ist. Die Anspritzkuppe 118 kann vorgesehen sein, wenn der Behälter 100 durch Spritzguss hergestellt worden ist.

Der Bodenabschnitt 110, z.B. die innere Oberfläche des Bodenabschnitts 110, kann eine Behälterachse 105 definieren. Die Behälterachse 105 kann sich senkrecht zu dem Bodenabschnitt 110 erstrecken. Der Bodenabschnitt 110 kann eine Ebene definieren, auf der die Behälterachse 105 senkrecht orientiert ist.

Die erste Wandseite 101 kann einen ersten Wandabschnitt 121, einen zweiten Wandabschnitt 131 und einen dritten Wandabschnitt 141 umfassen.

Der erste Wandabschnitt 121 kann sich (direkt) an den Bodenabschnitt 110 anschließen, insbesondere in Richtung der Behälterachse 105. Der zweite Wandabschnitt 131 kann sich (direkt) an den ersten Wandabschnitt anschließen, insbesondere in Richtung der Behälterachse 105. Der dritte Wandabschnitt 141 kann sich (direkt) an den zweiten Wandabschnitt 131 anschließen, insbesondere in Richtung der Behälterachse 105.

Zwischen der Behälterachse 105 und dem ersten Wandabschnitt 121 kann ein Winkel α121 gebildet sein. Der erste Wandabschnitt 121 kann um diesen Winkel α121 gegenüber der Behälterachse 105 geneigt sein. Bevorzugt ist der erste Wandabschnitt 121 gegenüber der Behälterachse 105 nach außen (vom Inneren des Behälters 100 wegweisend) geneigt.

Der Winkel α121 kann 1,9 ° ±0,7 ° oder ein n-faches davon betragen, wobei n ganzzahlig und größer 1 ist. Bevorzugt beträgt der Winkel α121 1,9 ° ±0,5 ° oder ein n-faches davon, wobei n ganzzahlig und größer 1 ist, bevorzugter 1,9 ° ±0,3 °, oder ein n-faches davon, wobei n ganzzahlig und größer 1 ist, bevorzugter 1,90 ° ±0,15 °, oder ein n-faches davon, wobei n ganzzahlig und größer 1 ist.

Ein Winkel α131 kann zwischen der Behälterachse 105 und dem zweiten Wandabschnitt 131 gebildet sein. Der zweite Wandabschnitt 131 kann um den Winkel α131 gegenüber der Behälterachse 105 (nach außen) geneigt sein.

Zwischen der Behälterachse 105 und dem dritten Wandabschnitt 141 kann ein Winkel α141 gebildet sein. Der dritte Wandabschnitt 141 kann um den Winkel α141 gegenüber der Behälterachse 105 (nach außen) geneigt sein.

Der erste Wandabschnitt 121, der zweite Wandabschnitt 131 und der dritte Wandabschnitt 141 können jeweils (nach außen) gegenüber der Behälterachse 105 geneigt sein.

Der Querschnitt (parallel zum Bodenabschnitt 110) des Behälters 100 kann am Bodenabschnitt 110 geringer sein als der Querschnitt des Behälters 100 im ersten Wandabschnitt 121 (auf mittlerer Höhe des ersten Wandabschnitts 121 in Richtung der Behälterachse 105). Der Querschnitt des Behälters 100 kann im ersten Wandabschnitt 121 (auf mittlerer Höhe des ersten Wandabschnitts 121 in Richtung der Behälterachse 105) geringer sein als der Querschnitt des Behälters 100 im zweiten Wandabschnitt 131 (auf mittlerer Höhe des zweiten Wandabschnitts 131 in Richtung der Behälterachse 105). Der Querschnitt des Behälters 100 kann im zweiten Wandabschnitt 131 (auf mittlerer Höhe des zweiten Wandabschnitts 131 in Richtung der Behälterachse 105) geringer sein als der Querschnitt des Behälters 100 im dritten Wandabschnitt 141 (auf mittlerer Höhe des dritten Wandabschnitts 141 in Richtung der Behälterachse 105).

Der Querschnitt des Behälters 100 kann sich in Richtung der Behälterachse 105 (kontinuierlich, bevorzugt mit nicht-konstanter Steigung) vergrößern. Durch die Ausgestaltung des Behälters 100 kann ein für eine Probenuntersuchung einzubringendes Flüssigkeitsvolumen (Clearingmittelvolumen) gering sein, sodass Flüssigkeit oder Clearingmittel gespart wird.

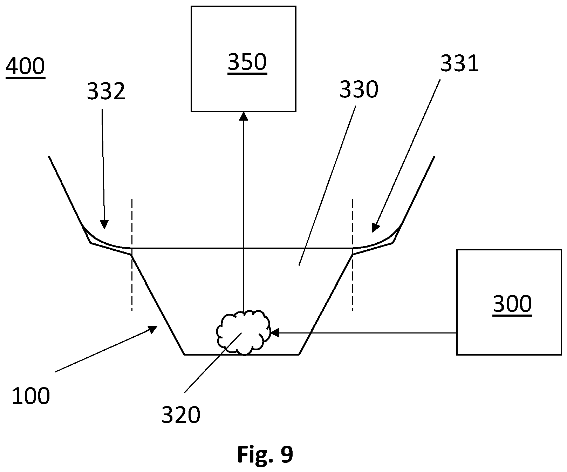

Ein Nutzer kann den Behälter 100 im oberen Bereich (dritter Wandabschnitt oder dritte Wandabschnitte) greifen. Dadurch werden Fingerabdrücke oder sonstige Oberflächenverunreinigungen an der Oberfläche des ersten Wandabschnitts oder der ersten Wandabschnitte reduziert oder vermieden. Der erste Wandabschnitt kann, wie mit Blick auf Figur 9 beschrieben, von einer Strahlungsquelle durchleuchtet werden.

Ebenso kann der obere Bereich (dritter Wandabschnitt oder dritte Wandabschnitte) des Behälters 100 mit einer Etikettierung versehen sein. Die Etikettierung kann einen Datamatrixcode und/oder eine fortlaufende Nummerierung umfassen. Die Etikettierung kann eine Information über den Behälter 100 und/oder über die darin zu lagernde oder darin lagernde Probe und/oder eine in den Behälter 100 einzubringende Flüssigkeit oder eingebrachte Flüssigkeit umfassen.

Zwischen dem ersten Wandabschnitt 121 und dem zweiten Wandabschnitt 131 kann ein Knick in der Neigung der Wandabschnitte 121, 131 gegenüber der Behälterachse 105 vorhanden sein. Der Knick kann sich durch eine sprunghafte oder diskontinuierliche Änderung der Neigung gegenüber der Behälterachse 105 ergeben. Zwischen dem zweiten Wandabschnitt 131 und dem dritten Wandabschnitt 141 kann ein Knick in der Neigung der Wandabschnitte 131, 141 gegenüber der Behälterachse vorhanden sein.

Der Knick zwischen dem ersten Wandabschnitt 121 und dem zweiten Wandabschnitt 131 und/oder der Knick zwischen dem zweiten Wandabschnitt 131 und dem dritten Wandabschnitt 141 kann als Markierung für eine vorgesehene Füllhöhe oder Füllmenge, beispielsweise eines Clearingmittels, des Behälters 100 dienen.

Der Winkel α121 zwischen der Behälterachse 105 und dem ersten Wandabschnitt 121 kann kleiner sein als der Winkel α131 zwischen der Behälterachse 105 und dem zweiten Wandabschnitt 131. Der Winkel α131 zwischen der Behälterachse 105 und dem zweiten Wandabschnitt 131 kann größer sein als der Winkel α141 zwischen der Behälterachse 105 und dem dritten Wandabschnitt 141. Der Winkel α121 zwischen der Behälterachse 105 und dem ersten Wandabschnitt 121 kann kleiner sein als der Winkel α141 zwischen der Behälterachse 105 und dem dritten Wandabschnitt 141.

Die dritte Wandseite 103 kann einen ersten Wandabschnitt 123, einen zweiten Wandabschnitt 133 und einen vierten Wandabschnitt 143 umfassen.

Der erste Wandabschnitt 123 der dritten Wandseite 103 kann sich (direkt) an den Bodenabschnitt 110 anschließen. Der zweite Wandabschnitt 133 der dritten Wandseite 103 kann sich (direkt) an den ersten Wandabschnitt 133 der dritten Wandseite 103 anschließen. Der dritte Wandabschnitt 143 der dritten Wandseite 103 kann sich (direkt) an den zweiten Wandabschnitt 133 der dritten Wandseite 103 anschließen.

Zwischen der Behälterachse 105 und dem ersten Wandabschnitt 123 der dritten Wandseite 103 kann ein Winkel α123 gebildet sein. Der erste Wandabschnitt 123 kann um diesen Winkel α123 gegenüber der Behälterachse 105 geneigt sein. Bevorzugt ist der erste Wandabschnitt 123 der dritten Wandseite 103 gegenüber der Behälterachse 105 nach außen (vom Inneren des Behälters 100 wegweisend) geneigt.

Ein Winkel α133 kann zwischen der Behälterachse 105 und dem zweiten Wandabschnitt 133 der dritten Wandseite 103 gebildet sein. Der zweite Wandabschnitt 133 der dritten Wandseite 103 kann um den Winkel α133 gegenüber der Behälterachse 105 (nach außen) geneigt sein.

Zwischen der Behälterachse 105 und dem dritten Wandabschnitt 143 kann ein Winkel α143 gebildet sein. Der dritte Wandabschnitt 143 der dritten Wandseite 103 kann um den Winkel α143 gegenüber der Behälterachse 105 (nach außen) geneigt sein.

Der erste Wandabschnitt 123 der dritten Wandseite 103, der zweite Wandabschnitt 131 der dritten Wandseite 103 und der dritte Wandabschnitt 141 der dritten Wandseite 103 können jeweils (nach außen) gegenüber der Behälterachse 105 geneigt sein.

Zwischen dem ersten Wandabschnitt 123 der dritten Wandseite 103 und dem zweiten Wandabschnitt 133 der dritten Wandseite 103 kann ein Knick in der Neigung der Wandabschnitte 123, 133 gegenüber der Behälterachse 105 vorhanden sein. Zwischen dem zweiten Wandabschnitt 133 der dritten Wandseite 103 und dem dritten Wandabschnitt 143 der dritten Wandseite 103 kann ein Knick in der Neigung der Wandabschnitte 133, 143 gegenüber der Behälterachse 105 vorhanden sein.

Der Winkel α123 zwischen der Behälterachse 105 und dem ersten Wandabschnitt 123 der dritten Wandseite 103 kann kleiner sein als der Winkel α133 zwischen der Behälterachse 105 und dem zweiten Wandabschnitt 133 der dritten Wandseite 103. Der Winkel α133 zwischen der Behälterachse 105 und dem zweiten Wandabschnitt 133 der dritten Wandseite 103 kann größer sein als der Winkel α143 zwischen der Behälterachse 105 und dem dritten Wandabschnitt 143 der dritten Wandseite 103. Der Winkel α123 zwischen der Behälterachse 105 und dem ersten Wandabschnitt 123 kann größer sein als der Winkel α143 zwischen der Behälterachse 105 und dem dritten Wandabschnitt 143 der dritten Wandseite 103.

Der erste Wandabschnitt 121 der ersten Wandseite 101 kann dem ersten Wandabschnitt 123 der dritten Wandseite 103 gegenüberliegen. Der erste Wandabschnitt 121 der ersten Wandseite 101 kann eine im Wesentlichen (±10 %) gleiche Höhe (in Richtung der Behälterachse 105) wie der erste Wandabschnitt 123 der dritten Wandseite 103 aufweisen.

Der zweite Wandabschnitt 131 der ersten Wandseite 101 kann dem zweiten Wandabschnitt 133 der dritten Wandseite 103 gegenüberliegen. Der zweite Wandabschnitt 131 der ersten Wandseite 101 kann eine im Wesentlichen (±10 %) gleiche Höhe wie der zweite Wandabschnitt 133 der dritten Wandseite 103 aufweisen.

Der dritte Wandabschnitt 141 der ersten Wandseite 101 kann dem dritten Wandabschnitt 143 der dritten Wandseite 103 gegenüberliegen. Der dritte Wandabschnitt 141 der ersten Wandseite 101 kann eine im Wesentlichen (±10 %) gleiche Höhe wie der dritte Wandabschnitt 143 der dritten Wandseite 103 aufweisen.

Der Winkel α121 zwischen der Behälterachse 105 und dem ersten Wandabschnitt 121 der ersten Wandseite 101 kann kleiner sein als der Winkel α123 zwischen der Behälterachse 105 und dem ersten Wandabschnitt 123 der dritten Wandseite 103.

Der Winkel α131 zwischen der Behälterachse 105 und dem zweiten Wandabschnitt 131 der ersten Wandseite 101 kann größer sein als der Winkel α123 zwischen der Behälterachse 105 und dem zweiten Wandabschnitt 133 der dritten Wandseite 103.

In Figur 4 ist der Schnitt B-B, wie in Figur 2 angedeutet, dargestellt. Der Bodenabschnitt 110, die zweite Wandseite 102 und die vierte Wandseite 104 sind geschnitten.

Die zweite Wandseite 102 kann einen ersten Wandabschnitt 122, einen zweiten Wandabschnitt 132 und einen dritten Wandabschnitt 142 umfassen.

Der erste Wandabschnitt 122 der zweiten Wandseite 102 kann sich (direkt) an den Bodenabschnitt 110 anschließen, insbesondere in Richtung der Behälterachse 105. Der zweite Wandabschnitt 132 der zweiten Wandseite 102 kann sich (direkt) an den ersten Wandabschnitt 122 der zweiten Wandseite 102 anschließen, insbesondere in Richtung der Behälterachse 105. Der dritte Wandabschnitt 142 der zweiten Wandseite 102 kann sich (direkt) an den zweiten Wandabschnitt 132 der zweiten Wandseite 102 anschließen, insbesondere in Richtung der Behälterachse 105.

Zwischen der Behälterachse 105 und dem ersten Wandabschnitt 122 der zweiten Wandseite 102 kann ein Winkel α122 gebildet sein. Der erste Wandabschnitt 122 der zweiten Wandseite 102 kann um diesen Winkel α122 gegenüber der Behälterachse 105 geneigt sein. Bevorzugt ist der erste Wandabschnitt 122 der zweiten Wandseite 102 gegenüber der Behälterachse 105 nach außen (vom Inneren des Behälters 100 wegweisend) geneigt.

Ein Winkel α132 kann zwischen der Behälterachse 105 und dem zweiten Wandabschnitt 132 der zweiten Wandseite 102 gebildet sein. Der zweite Wandabschnitt 132 der zweiten Wandseite 102 kann um den Winkel α132 gegenüber der Behälterachse 105 (nach außen) geneigt sein.

Zwischen der Behälterachse 105 und dem dritten Wandabschnitt 142 der zweiten Wandseite 102 kann ein Winkel α142 gebildet sein. Der dritte Wandabschnitt 142 der zweiten Wandseite 102 kann um den Winkel α142 gegenüber der Behälterachse 105 (nach außen) geneigt sein.

Der erste Wandabschnitt 122 der zweiten Wandseite 102, der zweite Wandabschnitt 132 der zweiten Wandseite 102 und der dritte Wandabschnitt 142 der zweiten Wandseite 102 können jeweils (nach außen) gegenüber der Behälterachse 105 geneigt sein.

Zwischen dem ersten Wandabschnitt 122 der zweiten Wandseite 102 und dem zweiten Wandabschnitt 132 der zweiten Wandseite 102 kann ein Knick in der Neigung der Wandabschnitte 122, 132 gegenüber der Behälterachse 105 vorhanden sein. Zwischen dem zweiten Wandabschnitt 132 der zweiten Wandseite 102 und dem dritten Wandabschnitt 142 der zweiten Wandseite 102 kann ein Knick in der Neigung der Wandabschnitte 132, 142 gegenüber der Behälterachse 105 vorhanden sein.

Der Winkel α122 zwischen der Behälterachse 105 und dem ersten Wandabschnitt 122 der zweiten Wandseite 102 kann kleiner sein als der Winkel α132 zwischen der Behälterachse 105 und dem zweiten Wandabschnitt 132 der zweiten Wandseite 102. Der Winkel α132 zwischen der Behälterachse 105 und dem zweiten Wandabschnitt 132 der zweiten Wandseite 102 kann größer sein als der Winkel α142 zwischen der Behälterachse 105 und dem dritten Wandabschnitt 142 der zweiten Wandseite 102. Der Winkel α122 zwischen der Behälterachse 105 und dem ersten Wandabschnitt 122 der zweiten Wandseite 102 kann kleiner sein als der Winkel α142 zwischen der Behälterachse 105 und dem dritten Wandabschnitt 142 der zweiten Wandseite 102.

Die vierte Wandseite 104 kann einen ersten Wandabschnitt 124, einen zweiten Wandabschnitt 134 und einen dritten Wandabschnitt 144 umfassen.

Der erste Wandabschnitt 124 der vierten Wandseite 104 kann sich (direkt) an den Bodenabschnitt 110 anschließen, insbesondere in Richtung der Behälterachse 105. Der zweite Wandabschnitt 134 der vierten Wandseite 104 kann sich (direkt) an den ersten Wandabschnitt 124 der vierten Wandseite 104 anschließen, insbesondere in Richtung der Behälterachse 105. Der dritte Wandabschnitt 144 der vierten Wandseite 104 kann sich (direkt) an den zweiten Wandabschnitt 134 der vierten Wandseite 104 anschließen, insbesondere in Richtung der Behälterachse 105.

Zwischen der Behälterachse 105 und dem ersten Wandabschnitt 124 der vierten Wandseite 104 kann ein Winkel α124 gebildet sein. Der erste Wandabschnitt 124 der vierten Wandseite 104 kann um diesen Winkel α124 gegenüber der Behälterachse 105 geneigt sein. Bevorzugt ist der erste Wandabschnitt 124 der vierten Wandseite 104 gegenüber der Behälterachse 105 nach außen (vom Inneren des Behälters 100 wegweisend) geneigt.

Ein Winkel α134 kann zwischen der Behälterachse 105 und dem zweiten Wandabschnitt 134 der vierten Wandseite 104 gebildet sein. Der zweite Wandabschnitt 134 der vierten Wandseite 104 kann um den Winkel α134 gegenüber der Behälterachse 105 (nach außen) geneigt sein.

Zwischen der Behälterachse 105 und dem dritten Wandabschnitt 144 der vierten Wandseite 104 kann ein Winkel α144 gebildet sein. Der dritte Wandabschnitt 144 der vierten Wandseite 104 kann um den Winkel α144 gegenüber der Behälterachse 105 (nach außen) geneigt sein.

Der erste Wandabschnitt 124 der vierten Wandseite 104, der zweite Wandabschnitt 134 der vierten Wandseite 104 und der dritte Wandabschnitt 144 der vierten Wandseite 104 können jeweils (nach außen) gegenüber der Behälterachse 105 geneigt sein.

Zwischen dem ersten Wandabschnitt 124 der vierten Wandseite 104 und dem zweiten Wandabschnitt 134 der vierten Wandseite 104 kann ein Knick in der Neigung der Wandabschnitte 124, 134 gegenüber der Behälterachse 105 vorhanden sein. Zwischen dem zweiten Wandabschnitt 134 der vierten Wandseite 104 und dem dritten Wandabschnitt 144 der vierten Wandseite 104 kann ein Knick in der Neigung der Wandabschnitte 134, 144 gegenüber der Behälterachse 105 vorhanden sein.

Der Winkel α124 zwischen der Behälterachse 105 und dem ersten Wandabschnitt 124 der vierten Wandseite 104 kann kleiner sein als der Winkel α134 zwischen der Behälterachse 105 und dem zweiten Wandabschnitt 134 der vierten Wandseite 104. Der Winkel α134 zwischen der Behälterachse 105 und dem zweiten Wandabschnitt 134 der vierten Wandseite 104 kann größer sein als der Winkel α144 zwischen der Behälterachse 105 und dem dritten Wandabschnitt 144 der vierten Wandseite 104. Der Winkel α124 zwischen der Behälterachse 105 und dem ersten Wandabschnitt 124 der vierten Wandseite 104 kann kleiner sein als der Winkel α144 zwischen der Behälterachse 105 und dem dritten Wandabschnitt 144 der vierten Wandseite 104.

Der Winkel α122 zwischen der Behälterachse 105 und dem ersten Wandabschnitt 122 der zweiten Wandseite 102 kann im Wesentlichen (±2 ° oder ±1 °) den gleichen Wert besitzen wie der Winkel α124 zwischen der Behälterachse 105 und dem ersten Wandabschnitt 124 der vierten Wandseite 104. Der Winkel α132 zwischen der Behälterachse 105 und dem zweiten Wandabschnitt 132 der zweiten Wandseite 102 kann im Wesentlichen (±2 ° oder ±1 °) den gleichen Wert besitzen wie der Winkel α134 zwischen der Behälterachse 105 und dem zweiten Wandabschnitt 134 der vierten Wandseite 104. Der Winkel α142 zwischen der Behälterachse 105 und dem dritten Wandabschnitt 142 der zweiten Wandseite 102 kann im Wesentlichen (±2 ° oder ±1 °) den gleichen Wert besitzen wie der Winkel α144 zwischen der Behälterachse 105 und dem dritten Wandabschnitt 144 der vierten Wandseite 104.

Der Winkel α122 zwischen der Behälterachse 105 und dem ersten Wandabschnitt 122 der zweiten Wandseite 102 kann größer sein als der Winkel α121 zwischen der Behälterachse 105 und dem ersten Wandabschnitt 121 der ersten Wandseite 101.

Der Winkel α124 zwischen der Behälterachse 105 und dem ersten Wandabschnitt 124 der vierten Wandseite 104 kann größer sein als der Winkel α121 zwischen der Behälterachse 105 und dem ersten Wandabschnitt 121 der ersten Wandseite 101.

Der Winkel α132 zwischen der Behälterachse 105 und dem zweiten Wandabschnitt 132 der zweiten Wandseite 102 kann kleiner sein als der Winkel α131 zwischen der Behälterachse 105 und dem zweiten Wandabschnitt 131 der ersten Wandseite 101.

Der Winkel α134 zwischen der Behälterachse 105 und dem zweiten Wandabschnitt 134 der vierten Wandseite 104 kann kleiner sein als der Winkel α131 zwischen der Behälterachse 105 und dem zweiten Wandabschnitt 131 der ersten Wandseite 101.

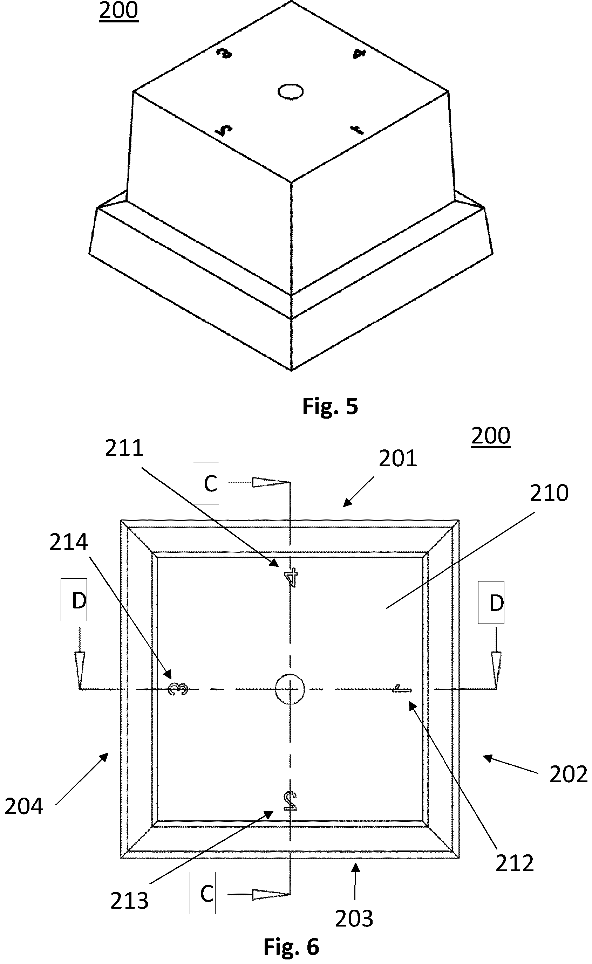

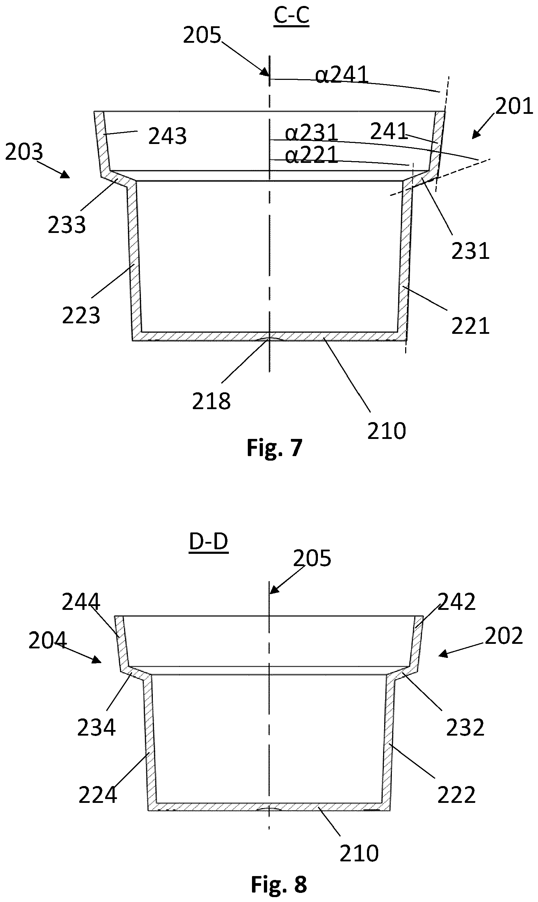

In den Figuren 5 und 6 ist ein Behälter 200 dargestellt. Der Behälter 200 ist in Figur 5 in einer isometrischen Ansicht gezeigt und in Figur 6 ist der Behälter 200 in einer Ansicht von unten gezeigt. In Figur 6 sind Schnittverläufe C-C und D-D angedeutet, die in den Figuren 7 und 8 dargestellt sind.

Der Behälter 200 kann einen Bodenabschnitt 210, eine erste Wandseite 201, eine zweite Wandseite 202, eine dritte Wandseite 203 und eine vierte Wandseite 204 umfassen.

Der Behälter 200 kann allgemein eine polygone Grundform aufweisen, bevorzugt weist der Bodenabschnitt 210 des Behälters 200 eine polygone Form auf. Die polygone Grundform oder Form kann quadratisch sein.

Der Behälter 200, insbesondere der Bodenabschnitt 210 des Behälters 200, kann Markierungen 211, 212, 213, 214 umfassen. Je eine der Markierungen 211, 212, 213, 214 kann je einer der Wandseiten 201, 202, 203, 204 zugeordnet sein. Die Markierung 211, 212, 213, 214 können der Identifikation der Wandseiten 201, 202, 203, 204 dienen. Beispielsweise können die Markierungen 211, 212, 213, 214 fortlaufende Nummern sein, z.B. 1, 2, 3, 4, oder die Markierungen 211, 212, 213, 214 können fortlaufende Buchstaben sein, z.B. A, B, C, D.

Die Figur 7 zeigt den Schnitt C-C, wobei der Bodenabschnitt 210, die erste Wandseite 201 und die dritte Wandseite 203 geschnitten sind.

Die innere Oberfläche (in das Innere des Behälters 200 gerichtet) des Bodenabschnitts 210 kann ebenflächig sein. Eine Anspritzkuppe 218 kann in dem Bodenabschnitt 210 so vorgesehen sein, dass trotz der Anspritzkuppe 218 die innere Oberfläche des Bodenabschnitts 210 ebenflächig ist. Die Anspritzkuppe 218 kann vorgesehen sein, wenn der Behälter 100 durch Spritzguss hergestellt worden ist.

Der Bodenabschnitt 210, z.B. die innere Oberfläche des Bodenabschnitts 210, kann eine Behälterachse 205 definieren. Die Behälterachse 205 kann sich senkrecht zu dem Bodenabschnitt 210 erstrecken. Der Bodenabschnitt 210 kann eine Ebene definieren, auf der die Behälterachse 205 senkrecht orientiert ist.

Die erste Wandseite 201 kann einen ersten Wandabschnitt 221, einen zweiten Wandabschnitt 231 und einen dritten Wandabschnitt 241 umfassen.

Der erste Wandabschnitt 221 kann sich (direkt) an den Bodenabschnitt 210 anschließen, insbesondere in Richtung der Behälterachse 205. Der zweite Wandabschnitt 231 kann sich (direkt) an den ersten Wandabschnitt 221 anschließen, insbesondere in Richtung der Behälterachse 205. Der dritte Wandabschnitt 241 kann sich (direkt) an den zweiten Wandabschnitt 231 anschließen, insbesondere in Richtung der Behälterachse 205.

Zwischen der Behälterachse 205 und dem ersten Wandabschnitt 221 kann ein Winkel a221 gebildet sein. Der erste Wandabschnitt 221 kann um diesen Winkel α221 gegenüber der Behälterachse 205 geneigt sein. Bevorzugt ist der erste Wandabschnitt 221 gegenüber der Behälterachse 205 nach außen (vom Inneren des Behälters 200 wegweisend) geneigt.

Ein Winkel α231 kann zwischen der Behälterachse 205 und dem zweiten Wandabschnitt 231 gebildet sein. Der zweite Wandabschnitt 231 kann um den Winkel α231 gegenüber der Behälterachse 205 (nach außen) geneigt sein.

Zwischen der Behälterachse 205 und dem dritten Wandabschnitt 241 kann ein Winkel α241 gebildet sein. Der dritte Wandabschnitt 241 kann um den Winkel α241 gegenüber der Behälterachse 205 (nach außen) geneigt sein.

Der erste Wandabschnitt 221, der zweite Wandabschnitt 231 und der dritte Wandabschnitt 241 können jeweils (nach außen) gegenüber der Behälterachse 205 geneigt sein.

Der Querschnitt (parallel zum Bodenabschnitt 210) des Behälters 200 kann am Bodenabschnitt 210 geringer sein als der Querschnitt des Behälters 200 im ersten Wandabschnitt 221 (auf mittlerer Höhe des ersten Wandabschnitts 221 in Richtung der Behälterachse 205). Der Querschnitt des Behälters 200 kann im ersten Wandabschnitt 221 (auf mittlerer Höhe des ersten Wandabschnitts 221 in Richtung der Behälterachse 205) geringer sein als der Querschnitt des Behälters 200 im zweiten Wandabschnitt 231 (auf mittlerer Höhe des zweiten Wandabschnitts 231 in Richtung der Behälterachse 205). Der Querschnitt des Behälters 200 kann im zweiten Wandabschnitt 231 (auf mittlerer Höhe des zweiten Wandabschnitts 231 in Richtung der Behälterachse 205) geringer sein als der Querschnitt des Behälters 200 im dritten Wandabschnitt 241 (auf mittlerer Höhe des dritten Wandabschnitts 241 in Richtung der Behälterachse 205).

Zwischen dem ersten Wandabschnitt 221 und dem zweiten Wandabschnitt 231 kann ein Knick in der Neigung der Wandabschnitte 221, 231 gegenüber der Behälterachse 205 vorhanden sein. Der Knick kann sich durch eine sprunghafte oder unkontinuierliche Änderung der Neigung gegenüber der Behälterachse 205 ergeben. Zwischen dem zweiten Wandabschnitt 231 und dem dritten Wandabschnitt 241 kann ein Knick in der Neigung der Wandabschnitte 231, 241 gegenüber der Behälterachse 205 vorhanden sein.

Der Winkel α221 zwischen der Behälterachse 205 und dem ersten Wandabschnitt 221 kann kleiner sein als der Winkel α231 zwischen der Behälterachse 205 und dem zweiten Wandabschnitt 231. Der Winkel α231 zwischen der Behälterachse 205 und dem zweiten Wandabschnitt 231 kann größer sein als der Winkel α241 zwischen der Behälterachse 205 und dem dritten Wandabschnitt 241. Der Winkel α221 zwischen der Behälterachse 205 und dem ersten Wandabschnitt 221 kann kleiner sein als der Winkel α241 zwischen der Behälterachse 205 und dem dritten Wandabschnitt 241.

Die dritte Wandseite 203 kann einen ersten Wandabschnitt 223, einen zweiten Wandabschnitt 233 und einen vierten Wandabschnitt 243 umfassen.

Der erste Wandabschnitt 223 der dritten Wandseite 203 kann sich (direkt) an den Bodenabschnitt 210 anschließen. Der zweite Wandabschnitt 233 der dritten Wandseite 203 kann sich (direkt) an den ersten Wandabschnitt 233 der dritten Wandseite 203 anschließen. Der dritte Wandabschnitt 243 der dritten Wandseite 203 kann sich (direkt) an den zweiten Wandabschnitt 233 der dritten Wandseite 203 anschließen.

Der erste Wandabschnitt 221 der ersten Wandseite 201 kann dem ersten Wandabschnitt 223 der dritten Wandseite 203 gegenüberliegen. Der erste Wandabschnitt 221 der ersten Wandseite 201 kann eine im Wesentlichen (±10 %) gleiche Höhe (in Richtung der Behälterachse 205) wie der erste Wandabschnitt 223 der dritten Wandseite 203 aufweisen.

Der zweite Wandabschnitt 231 der ersten Wandseite 201 kann dem zweiten Wandabschnitt 233 der dritten Wandseite 203 gegenüberliegen. Der zweite Wandabschnitt 231 der ersten Wandseite 201 kann eine im Wesentlichen (±10 %) gleiche Höhe wie der zweite Wandabschnitt 233 der dritten Wandseite 203 aufweisen.

Der dritte Wandabschnitt 241 der ersten Wandseite 201 kann dem dritten Wandabschnitt 243 der dritten Wandseite 203 gegenüberliegen. Der dritte Wandabschnitt 241 der ersten Wandseite 201 kann eine im Wesentlichen (±10 %) gleiche Höhe wie der dritte Wandabschnitt 243 der dritten Wandseite 203 aufweisen.

Die Neigung des ersten Wandabschnitts 223 der dritten Wandseite 203 kann der Neigung des ersten Wandabschnitts 221 der ersten Wandseite 201 entsprechen oder gleich dazu sein. Die Neigung des zweiten Wandabschnitts 233 der dritten Wandseite 203 kann der Neigung des zweiten Wandabschnitts 231 der ersten Wandseite 201 entsprechen oder gleich dazu sein. Die Neigung des dritten Wandabschnitts 243 der dritten Wandseite 203 kann der Neigung des dritten Wandabschnitts 241 der ersten Wandseite 201 entsprechen oder gleich dazu sein.

Allgemein kann die dritte Wandseite 203 der ersten Wandseite 201 so entsprechen, dass die dritte Wandseite 203 eine Spiegelung der ersten Wandseite 201 gegenüber der Behälterachse 205 ist. Die Behälterachse 205 kann allgemein in der Mitte des Bodenabschnitts 210 gebildet sein.

Figur 8 zeigt den Schnitt D-D, wie in Figur 6 angedeutet, wobei der Bodenabschnitt 210, die zweite Wandseite 202 und die vierte Wandseite 204 geschnitten sind.

Die zweite Wandseite 202 kann einen ersten Wandabschnitt 222, einen zweiten Wandabschnitt 232 und einen dritten Wandabschnitt 242 umfassen.

Der erste Wandabschnitt 222 der zweiten Wandseite 202 kann die Merkmale des ersten Wandabschnitts 221 der ersten Wandseite 201 aufweisen. Der zweite Wandabschnitt 232 der zweiten Wandseite 202 kann die Merkmale des zweiten Wandabschnitts 231 der ersten Wandseite 201 aufweisen. Der dritte Wandabschnitt 242 der zweiten Wandseite 202 kann die Merkmale des dritten Wandabschnitts 241 der ersten Wandseite 201 aufweisen.

Die vierte Wandseite 204 kann einen ersten Wandabschnitt 224, einen zweiten Wandabschnitt 234 und einen dritten Wandabschnitt 244 umfassen.

Der erste Wandabschnitt 224 der vierten Wandseite 204 kann die Merkmale des ersten Wandabschnitts 221 der ersten Wandseite 201 aufweisen. Der zweite Wandabschnitt 234 der vierten Wandseite 204 kann die Merkmale des zweiten Wandabschnitts 231 der ersten Wandseite 201 aufweisen. Der dritte Wandabschnitt 244 der vierten Wandseite 204 kann die Merkmale des dritten Wandabschnitts 241 der ersten Wandseite 201 aufweisen.

Allgemein kann die erste Wandseite 201 des Behälters 200 die Merkmale der ersten Wandseite 101 des Behälters 100 aufweisen. Die zweite Wandseite 202 des Behälters 200 kann die Merkmale der zweiten Wandseite 102 des Behälters 100 aufweisen. Die dritte Wandseite 203 des Behälters 200 kann die Merkmale der dritten Wandseite 103 des Behälters 100 aufweisen. Die vierte Wandseite 204 des Behälters 200 kann die Merkmale der vierten Wandseite 104 des Behälters 100 aufweisen. Analog kann jede der Wandseiten 101, 102, 103, 104 des Behälters 100 die Merkmale jeder der Wandseiten 201, 202, 203, 204 des Behälters 200 aufweisen.

Allgemein kann ein Winkel oder eine Neigung, der oder die größer als ein anderer Winkel oder eine andere Neigung ist, um zumindest 1 °, 2 °, 3 ° oder 5 ° größer sein. Allgemein kann ein Winkel oder eine Neigung, der oder die kleiner als ein anderer Winkel oder eine andere Neigung ist, um zumindest 1 °, 2 °, 3 ° oder 5 ° kleiner sein.

Figur 9 zeigt ein System 400 zum Messen einer Probe 320. Die Probe 320 kann in einen Behälter 100 eingebracht sein. In Figur 9 ist exemplarisch ein Behälter 100 angedeutet, ein Behälter 200 kann ebenso verwendet werden.

Die Probe 320 kann eine Höhe (in Richtung der Behälterachse) von mindestens 2 mm, bevorzugt mindestens 5 mm, bevorzugter mindestens 10 mm, bevorzugter mindestens 20 mm, aufweisen.

In den Behälter 100 kann eine Flüssigkeit 330 eingebracht sein. Die Flüssigkeit 330 kann die Probe 320 vollständig bedecken. Die Oberfläche der Flüssigkeit 330 kann in einem Kontaktbereich mit einer Wand oder mit Wänden des Behälters 100 gewölbt sein. In dem Kontaktbereich kann sich ein Meniskus 331, 332 bilden. Die Flüssigkeit 330 kann so in den Behälter 100 eingebracht sein, dass die Oberfläche der Flüssigkeit 330 parallel zu einer Querschnittsfläche des ersten Wandabschnitts oder der ersten Wandabschnitte des Behälters 100 im Wesentlichen (± 5 °) ebenflächig ist. Dabei kann die Querschnittsfläche des ersten Wandabschnitts oder der ersten Wandabschnitte des Behälters auf die Oberfläche der Flüssigkeit 330 projiziert sein. Es kann also eine Fläche an der Oberfläche der Flüssigkeit 330 betrachtet werden, die die gleichen Maße wie die Querschnittsfläche des ersten Wandabschnitts besitzt.

Das System kann eine Strahlungsquelle 300 umfassen. Die Strahlungsquelle 300 kann ein Laser sein. Durch die Strahlungsquelle 300 kann die Probe 320 beleuchtet werden. Bevorzugt wird die Probe 320 von der Strahlungsquelle 300 durch einen ersten Wandabschnitt des Behälters 100 beleuchtet. Durch die Beleuchtung kann eine Emission der Probe 320 angeregt werden. Die Beleuchtung der Probe 320 kann durch eine der Wandseiten erfolgen, durch zumindest zwei der Wandseiten erfolgen, oder durch alle Wandseiten des Behälters erfolgen. Die Beleuchtung durch unterschiedliche Wandseiten kann nacheinander erfolgen. Gleichzeitig kann durch höchstens eine oder zwei Wandseiten beleuchtet werden.

Das System 400 kann einen Strahlungsempfänger 350 umfassen. Der Strahlungsempfänger 350 kann eine Kamera sein. Der Strahlungsempfänger 350 kann die Emission der Probe 320 aufnehmen. Bevorzugt liegen die Strahlungsquelle 300, die Probe 320 und der Strahlungsempfänger 350 nicht auf einer (geraden) Linie. Der Strahlungsempfänger 350 kann so angeordnet sein, dass eine Emission der Probe 320 im Wesentlichen (±10°) senkrecht zur Beleuchtungsrichtung der Probe 320 durch die Strahlungsquelle 300 aufnehmbar ist oder aufgenommen wird. Der Strahlungsempfänger 350 kann so angeordnet sein, dass eine Emission der Probe 320 im Wesentlichen (±10°) senkrecht zum Bodenabschnitt des Behälters 100 aufnehmbar ist oder aufgenommen wird.

Zwischen der Probe 320 und dem Strahlungsempfänger 350 kann ein Objektiv angeordnet sein.

Das Objektiv kann während der Beleuchtung oder Messung der Probe 320 zumindest teilweise in die Flüssigkeit 330 eingetaucht sein. Das Objektiv kann auch während der Beleuchtung der Probe 320 oberhalb der Flüssigkeitsoberfläche positioniert sein.

Eine Öffnung des Behälters 100 kann während der der Beleuchtung der Probe 320 bedeckt sein, beispielsweise von einer oder mehreren Glasplatten.

Beispielsweise kann die Strahlungsquelle 300 die Probe 320 durch einen ersten Wandabschnitt des Behälters 100 hindurch beleuchten. Da die erste Wandseite eine Neigung von 1,9 ° ±0,7 ° oder ein n-faches davon aufweist und durch die Brechungsindizes der Grenzflächen auf dem Weg der Strahlung von der Strahlungsquelle 300 zu der Probe 320, kann die Strahlung der Strahlungsquelle 330 gradweise (ganzzahlig) so korrigiert werden, dass die Strahlung parallel zum Bodenabschnitt oder parallel zu einer Flüssigkeitsoberfläche, wenn die Probe 320 von einer Flüssigkeit bedeckt ist, auf die Probe 320 auftrifft. Gleichzeitig lässt sich der Behälter kostengünstig in einem Spritzgussverfahren herstellen (Entformungsschrägen).

Ist der erste Wandabschnitt der dritten Wandseite des Behälters 100 stärker geneigt als der erste Wandabschnitt der Wandseite, durch den die Beleuchtung erfolgt, können störende Reflexionen vermieden werden. Speziell bei einer kleinen Probe, die von der Beleuchtung der Strahlungsquelle 330 vollständig durchdrungen wird, kann Strahlung in Richtung der Probe 330 reflektiert werden, wenn der erste Wandabschnitt der dritten Wandseite nicht stärker geneigt ist als der erste Wandabschnitt der Wandseite, durch den die Beleuchtung erfolgt.

Die rechteckige (nicht-quadratische) Grundform des Behälters 100 oder des Bodenabschnitts 110 des Behälters 100 kann dem optimalen, dünnen Teil (Rayleigh-Länge) eines Lichtblatts (Strahlungsquelle 300) entlang der Beleuchtungsrichtung und der Feldgröße eines Objektivs in senkrechter Richtung zu der Beleuchtungsrichtung entsprechen.

## Patentansprüche

1. Behälter (100, 200) für eine Probe (320), der Behälter (100, 200) mit:
einem Bodenabschnitt (110, 210), wobei eine Behälterachse (105, 205) senkrecht zu dem Bodenabschnitt (110, 210) orientiert ist;
einem ersten Wandabschnitt (121, 221), wobei sich der erste Wandabschnitt (121, 221) an den Bodenabschnitt (110, 210) anschließt; und
einem zweiten Wandabschnitt (131, 231), wobei sich der zweite Wandabschnitt (131, 231) an den ersten Wandabschnitt (110, 210) anschließt, und wobei der zweite Wandabschnitt (131, 231) gegenüber der Behälterachse (105, 205) um zumindest 20 ° geneigt ist.

2. Behälter (100, 200) nach Anspruch 1, wobei der erste Wandabschnitt (121, 221) gegenüber der Behälterachse (105, 205) um zumindest 0,5 ° geneigt ist, bevorzugt um zumindest 1,0 ° geneigt ist, besonders bevorzugt um zumindest 1,5 °, geneigt ist; und/oder wobei der erste Wandabschnitt (121, 221) gegenüber der Behälterachse (105, 205) um höchstens 25 ° geneigt ist, bevorzugt um höchstens 10 ° geneigt ist, besonders bevorzugt um höchstens 5 °, geneigt ist.

3. Behälter (100, 200) nach einem der Ansprüche 1 oder 2, wobei der Behälter (100, 200) einen dritten Wandabschnitt (141, 241) umfasst, wobei sich der dritte Wandabschnitt (141, 241) an den zweiten Wandabschnitt (131, 231) anschließt und wobei der dritte Wandabschnitt (141, 241) gegenüber der Behälterachse (105, 205) um zumindest 2 ° geneigt ist.

4. Behälter (100, 200) nach einem der vorhergehenden Ansprüche, wobei der Behälter (100, 200) eine polygone, insbesondere rechteckige oder quadratische, Grundform aufweist und wobei der Behälter (100, 200) eine erste Wandseite (101, 201), eine zweite Wandseite (102, 202), eine dritte Wandseite (103, 203) und eine vierte Wandseite (104, 204) umfasst.

5. Behälter (100) nach Anspruch 4, wobei die erste Wandseite (101) den ersten, zweiten und dritten Wandabschnitt (121, 131, 141) umfasst, wobei die dritte Wandseite (103) einen ersten, zweiten und dritten Wandabschnitt (123, 133, 143) umfasst, und wobei der erste Wandabschnitt (123) der dritten Wandseite (103) eine größere Neigung gegenüber der Behälterachse (105) aufweist als der erste Wandabschnitt (121) der ersten Wandseite (101), insbesondere wobei der erste Wandabschnitt (123) der dritten Wandseite (103) eine um zumindest 2 °, bevorzugt zumindest 5 °, größere Neigung gegenüber der Behälterachse (105) aufweist als der erste Wandabschnitt (121) der ersten Wandseite (101).

6. Behälter (100) nach einem der Ansprüche 4 oder 5, wobei die erste Wandseite (101) den ersten, zweiten und dritten Wandabschnitt (121, 131, 141) umfasst, wobei die dritte Wandseite (103) einen ersten, zweiten und dritten Wandabschnitt (123, 133, 143) umfasst, und wobei der zweite Wandabschnitt (133) der dritten Wandseite (103) eine geringere Neigung gegenüber der Behälterachse (105) aufweist als der zweite Wandabschnitt (131) der ersten Wandseite (101), insbesondere wobei der zweite Wandabschnitt (133) der dritten Wandseite (103) eine um zumindest 2 °, bevorzugt zumindest 5 °, geringere Neigung gegenüber der Behälterachse (105) aufweist als der zweite Wandabschnitt (131) der ersten Wandseite (101).

7. Behälter (100) nach einem der Ansprüche 4 bis 6, wobei die erste Wandseite (101) den ersten, zweiten und dritten Wandabschnitt (121, 131, 141) umfasst, wobei die zweite und/oder vierte Wandseite (102, 104) jeweils einen ersten, zweiten und dritten Wandabschnitt (122, 124, 132, 134, 142, 144) umfasst, und wobei der erste Wandabschnitt (122, 124) der zweiten Wandseite (102) und/oder der vierten Wandseite (104) eine größere Neigung gegenüber der Behälterachse (105) aufweist als der erste Wandabschnitt (121) der ersten Wandseite (101), insbesondere wobei der erste Wandabschnitt (122, 124) der zweiten Wandseite (102) und/oder der vierten Wandseite (104) eine um zumindest 1 °, bevorzugt zumindest 2 °, größere Neigung gegenüber der Behälterachse (105) aufweist als der erste Wandabschnitt (121) der ersten Wandseite (101).

8. Behälter (100) nach einem der Ansprüche 4 bis 7, wobei die erste Wandseite (101) den ersten, zweiten und dritten Wandabschnitt (121, 131, 141) umfasst, wobei die zweite und/oder vierte Wandseite (102, 104) jeweils einen ersten, zweiten und dritten Wandabschnitt (122, 124, 132, 134, 142, 144) umfasst, und wobei der zweite Wandabschnitt (132, 134) der zweiten Wandseite (102) und/oder der vierten Wandseite (104) eine geringere Neigung gegenüber der Behälterachse (105) aufweist als der zweite Wandabschnitt (131) der ersten Wandseite (101), insbesondere wobei der zweite Wandabschnitt (132, 134) der zweiten Wandseite (102) und/oder der vierten Wandseite (104) eine um zumindest 1 °, bevorzugt zumindest 2 °, geringere Neigung gegenüber der Behälterachse (105) aufweist als der zweite Wandabschnitt (131) der ersten Wandseite (101).

9. Behälter (200) nach Anspruch 4, wobei jede der ersten, zweiten, dritten und vierten Wandseiten (201, 202, 203, 204) den ersten Wandabschnitt (221) und/oder den zweiten Wandabschnitt (231) und/oder den dritten Wandabschnitt (241) umfasst.

10. Behälter (100, 200) für eine Probe (320), der Behälter (100, 200) mit:
einem Bodenabschnitt (110, 210), wobei eine Behälterachse (105, 205) senkrecht zu dem Bodenabschnitt (110, 210) orientiert ist; und
einem ersten Wandabschnitt (121, 221), wobei sich der erste Wandabschnitt (121, 221) an den Bodenabschnitt (110, 210) anschließt und wobei der erste Wandabschnitt (121, 221) gegenüber der Behälterachse (105, 205) um 1,9 ° ±0,7 ° oder ein n-faches davon, wobei n ganzzahlig und größer 1 ist, geneigt ist.

11. Behälter (100, 200) nach Anspruch 10, wobei der erste Wandabschnitt (121, 221) gegenüber der Behälterachse (105, 205) um 1,9 ° ±0,5 °, insbesondere um 1,9 ° ±0,3 °, oder ein n-faches davon, wobei n ganzzahlig und größer 1 ist, geneigt ist.

12. Behälter (100, 200) nach einem der vorhergehenden Ansprüche, wobei der Behälter (100, 200) ein spritzgegossener Behälter (100, 200) ist, und/oder der Behälter (100, 200) einen Thermoplast umfasst, bevorzugt aus einem Thermoplast besteht und/oder wobei der Behälter (100, 200) ein Block-Copolymer, insbesondere ein cyclisches Block-Copolymer, umfasst oder daraus besteht.

13. Behälter (100, 200) nach einem der vorhergehenden Ansprüche, wobei zumindest ein Abschnitt des Behälters (100, 200) aus einem Material besteht, wobei das Material eine oder mehrere der folgenden Eigenschaften aufweist:
eine Dichte, bestimmt nach ASTM D792, von höchstens 1,00 g cm⁻³, bevorzugt von höchstens 0,98 g cm⁻³, bevorzugter von höchstens 0,96 g cm⁻³, besonders bevorzugt zwischen 0,92 g cm⁻³ und 0,96 g cm⁻³; und/oder
eine Wasseraufnahme, bestimmt nach ASTM D570, von weniger als 5 %, bevorzugt von weniger als 2,0 %, bevorzugter von weniger als 0,5 %; und/oder
eine Schmelzflussrate, bestimmt nach ASTM D1238 bei 2,6 kg und 260 °C, von zumindest 1,0 cm³ 10 min⁻¹, bevorzugt von zumindest 7,0 cm³ 10 min⁻¹, bevorzugter von zumindest 100,0 cm³ 10 min⁻¹, bevorzugter zwischen 2,0 cm³ 10 min⁻¹ und 250,0 cm³ 10 min⁻¹; und/oder
eine Transmission, bestimmt nach ASTM D1003, 3 mm, von zumindest 50 %, bevorzugt zumindest 70 %, bevorzugter zumindest 85 %, bevorzugter zumindest 90 %; und/oder
eine Trübung, bestimmt nach ASTM D1003, 3 mm, von weniger als 5,0 %, bevorzugt von weniger als 3,0%, bevorzugter von weniger als 1,5%, bevorzugter von weniger als 1,0 %; und/oder
einen Reflexionsindex von weniger als 5,0, bevorzugt von weniger als 3,0, bevorzugter von weniger als 2,5; und/oder
eine Vicat-Erweichungstemperatur, bestimmt nach ASTM D1525, 1 kg, 50 °C h⁻¹, von zumindest 70 °C, bevorzugt zumindest 85 °C, bevorzugter zumindest 100 °C, bevorzugter zwischen 90 °C und 130 °C; und/oder
eine Wärmeformbeständigkeitstemperatur, bestimmt nach ASTM D648, 0,455 MPa, 2 °C min⁻¹, von zumindest 50 °C, bevorzugt zumindest 60 °C, bevorzugter zumindest 75 °C, bevorzugter zwischen 60 °C und 115 °C; und/oder
eine Biegefestigkeit, bestimmt nach ASTM D790, von zumindest 30 MPa, bevorzugt zumindest 40 MPa, bevorzugter zumindest 50 MPa, bevorzugter zwischen 40 MPa und 80 MPa; und/oder
ein Biegemodul, bestimmt nach ASTM D790, von zumindest 600 MPa, bevorzugt zumindest 1000 MPa, bevorzugter zumindest 1400 MPa, bevorzugter zwischen 1000 MPa und 3000 MPa; und/oder
eine Zugfestigkeit, Y.P. oder B.P., bestimmt nach ASTM D638, von zumindest 15 MPa, bevorzugt zumindest 25 MPa, bevorzugter zumindest 30 MPa, bevorzugter zwischen 20 MPa und 50 MPa; und/oder
eine Dehnung, bestimmt nach ASTM D638, von zumindest 5 %, bevorzugt zumindest 8 %, bevorzugter zumindest, 10 %, bevorzugter zwischen 8 % und 40 %; und/oder
eine Izod-Schlagzähigkeit, bestimmt nach ASTM D256, von zumindest 1 kg-cm cm⁻¹, bevorzugt zumindest 1,5 kg-cm cm⁻¹, bevorzugter zumindest 2,0 kg-cm cm⁻¹, bevorzugter zwischen 1,5 kg-cm cm⁻¹ und 5,0 kg-cm cm⁻¹; und/oder
eine chemische Beständigkeit gegenüber Methylsalicylat, Dibenzylether, einer Mischung aus einem Teil Benzylalkohol und zwei Teilen Benzylbenzoat, einer Mischung aus vier Teilen Benzylbenzoat und einem Teil Diphenylether, Ethylcinnamat, und/oder 2,2'-Thiodiethanol; und/oder
eine Autofluoreszenz zwischen 400 nm und 460 nm, bei einer Anregung bei 350 nm und einer Probendicke von 1 mm, bezogen auf die Autofluoreszenz von Polystyrol von höchsten 50 %, bevorzugt höchstens 40 %, bevorzugter höchstens 30 %, bevorzugter höchstens 25 %.

14. Verfahren zur Untersuchung einer Probe (320), insbesondere einer biologischen Probe, mit den Schritten:
Platzieren der Probe (320) in einen Behälter (100, 200) nach einem der vorhergehenden Ansprüche;
Beleuchten der Probe (320); und
Aufnehmen einer Emission der Probe.

15. Verwendung eines Behälters (100, 200) nach einem der Ansprüche 1 bis 13 für eine Lagerung einer Probe (320) während einer Untersuchung der Probe (320), bevorzugt während einer mikroskopischen Untersuchung, bevorzugter während einer lichtscheibenmikroskopischen Untersuchung, bevorzugter während einer lichtscheibenfluoreszenzmikroskopischen Untersuchung.
